# EUROPEAN PATENT APPLICATION

(11) **EP 4 253 537 A1**
(43) Date of publication of application: **04.10.2023**
(21) Application number: 21897809.6
(22) Date of filing: 17.11.2021
(51) Int. Cl.: C12N 15/09, C12M 1/00, C12Q 1/6876

(54) **METHOD OF DESIGNING PRIMER FOR AMPLICON METHYLATION SEQUENCE ANALYSIS, PRODUCTION METHOD, DESIGNING DEVICE, DESIGNING PROGRAM AND RECORDING MEDIUM**

(30) Priority: 26.11.2020 JP 2020195943
(71) Applicant: FUJIFILM Corporation, Tokyo 106-8620 (JP)
(72) Inventor: YAMAGUCHI, Naoko, Ashigarakami-gun, Kanagawa 258-8538 (JP)
(74) Representative: Owen, Deborah Jane
(86) International application number: PCT/JP2021/042153
(87) International publication number: WO 2022/113835

(57) **Abstract**

An object of the present invention is to provide a design method, a manufacturing method, a design device, a design program, and a recording medium of a primer for amplicon methylation sequence analysis, which can improve a design success rate of the primer. The present invention is a primer design method for amplicon methylation sequence analysis, the method having a base conversion step of converting methylatable "C" into "Y" and converting other "C" into "T" in double-stranded genomic DNA, and a primer candidate sequence selection step of selecting sequences satisfying predetermined selection conditions as primer candidate sequences, in which the methylatable C is C in a CG sequence, and the predetermined selection conditions include (1) a Tm value is within a predetermined range, (2) the number of YG sequences or CR sequences included in a partial sequence is equal to or less than a predetermined, and (3) an upper limit of the number of binding sites with a sequence outside the related region on the double-stranded genomic DNA after base conversion is equal to or less than a predetermined number that is 1 or more.

## Description

### BACKGROUND OF THE INVENTION

### 1. Field of the Invention

The present invention relates to a design method, a manufacturing method, a design device, a design program, and a recording medium for a primer for amplicon methylation sequence analysis. Particularly, the present invention relates to a primer design method for designing a primer for simultaneously amplifying a plurality of amplification target regions including a plurality of target sites in deoxyribonucleic acid (DNA) treated with bisulfite or an enzyme by a multiplex polymerase chain reaction (PCR) and a manufacturing method, a design device, a design program, and a recording medium for the primer.

### 2. Description of the Related Art

DNA methylation is known as one of the epigenetic mechanisms, which is a gene expression control mechanism that is not involved in changes in DNA base sequence. Mammalian DNA methylation occurs mainly at the 5-position carbon atom of cytosine (C) in a CG sequence on DNA.

Gene promoter regions have a lot of regions called CpG islands where the CG sequence appear with high frequency. It is known that many CG sequences in these regions are unmethylated initially, but they are methylated due to diseases, development, differentiation, inflammation, aging, and the like and suppress gene expression. For example, it is known that in cancer cells, many of cancer suppressor gene groups are inactivated due to the acceleration of methylation of the CpG islands in a gene promoter region.

As described above, DNA methylation is highly involved in the control of gene expression. Therefore, the information on DNA methylation is considered to be useful for clarification of the mechanism of a disease such as cancer, evaluation of the differentiation status of various cells, and the like and is drawing attention in various fields such as diagnosis, treatment, drug discovery, and regenerative medicine, and research and development are actively carried out for the DNA methylation. For example, the DNA methylation status of a specific region is measured and analyzed to make an attempt to investigate whether or not different types of cells have drug resistance in developing drugs, an attempt to evaluate the presence or absence of cancer cells or malignancy (progress) of cancer cells based on the ratio between normal cells and abnormal cells, and an attempt to evaluate the differentiation status of stem cells and use the evaluation result for quality control of the stem cells.

As one of the methods of analyzing the DNA methylation status, there is a method using a bisulfite (hydrogen sulfite) reaction.

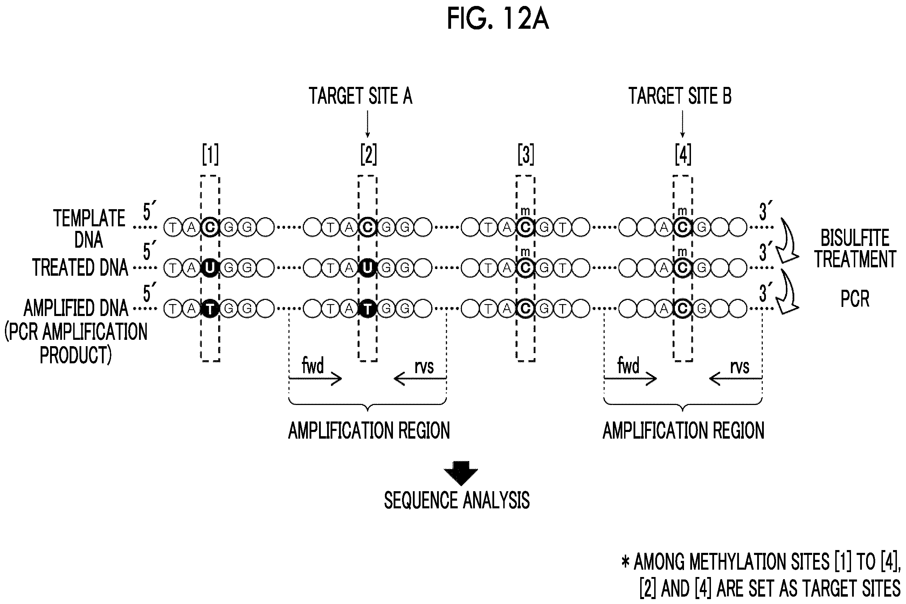

For example, cytosine (C) in a CG sequence related to a certain disease is picked up and adopted as a target site (measurement site). In Fig. 12A, [1] to [4] are methylation sites, and among these sites, [2] and [4] are set as target sites A and B (Fig. 12A shows only one strand).

Subsequently, a template DNA is treated with bisulfite (hydrogen sulfite). In a case where cytosine (C) in the CG sequence is methylated on the template DNA, cytosine (C) remains as it is after the treatment (see the methylation sites [3] and [4] in Fig. 12A). On the other hand, in a case where cytosine (C) in the CG sequence is unmethylated on the template DNA, cytosine (C) is deaminated and converted into uracil (U) (see methylation sites [1] and [2] in Fig. 12A).

Recently, instead of the bisulfite treatment, a method has been used which is a method of performing base conversion similar to the aforementioned reaction by using, for example, an enzyme such as NEB Next Enzymatic Methyl-seq Kit manufactured by New England Biolabs.

Then, for sequence analysis, the bisulfite-treated DNA is amplified using a polymerase chain reaction (PCR). The amplified DNA, that is, the PCR amplification product is subjected to sequence analysis using a capillary sequencer or a next generation sequencer (NGS).

In a case where the bisulfite-treated DNA is amplified using PCR, cytosine (C) remains as it is, (see the methylation sites [3] and [4] in Fig. 12A), whereas uracil (U) is replaced with thymine (T) and amplified (see the methylation sites [1] and [2] in Fig. 12A).

For example, utilizing the difference between cytosine (C) and thymine (T) caused in the sequence of the PCR amplification product makes it possible to ascertain the methylation status of a predetermined target site in DNA before the bisulfite treatment (template DNA), that is, to detect whether or not DNA of a predetermined target site selected from one cell is methylated. More specifically, based on whether a base in a predetermined target site of a PCR amplification product is cytosine (C) or thymine (T), it is possible to ascertain whether cytosine (C) in the predetermined target site of a template DNA is methylated or unmethylated. As shown in Fig. 12A, the base in a target site A of the PCR amplification product is thymine (T), which tells that cytosine (C) in the target site A of the template DNA is unmethylated. On the other hand, the base of the PCR amplification product of a target site B is cytosine (C), which tells that cytosine (C) in the target site B of the template DNA is methylated.

In addition, utilizing the difference between cytosine (C) and thymine (T) caused in the sequence of the PCR amplification product makes it possible to detect the methylation status (frequency) of bisulfite-untreated DNA (template DNA) of a specific target site derived from a plurality of cells, that is, to detect whether or not the DNA of a specific target site derived from a plurality of cells is methylated, and also makes it possible to ascertain the proportion of cells in which DNA methylation has occurred in a specific target site based on the detection result. In a case where there is a plurality of specific target sites, by detecting whether or not DNA methylation has occurred in each of the specific target sites, it is possible to detect the proportion of cells in which DNA methylation has occurred for each of the target sites based on the detection result. More specifically, based on whether the base in the specific target sites is cytosine (C) or thymine (T), it is possible to ascertain the DNA methylation status (frequency) of the specific target sites derived from a plurality of cells. The DNA methylation status (frequency) of the specific target sites can be obtained by calculating Methylation degree = C/(C + T) based on the number of cytosine (C) and thymine (T) generated in each target site (measurement site). In a case where there is a plurality of specific target sites, the proportion of cells in which DNA methylation has occurred can be ascertained for each of the specific target sites.

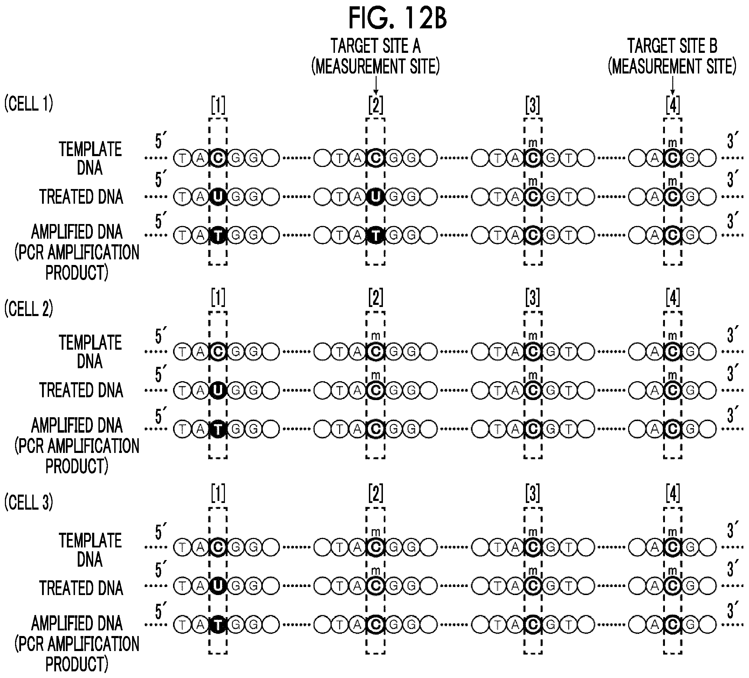

For example, as shown in Fig. 12B, in a case where a plurality of cells (cells 1 to 3 in Fig. 12B) is used to evaluate methylation status (frequency) of target sites (measurement sites) A and B derived from the plurality of cells, the number of cytosine (C) generated in the target site A is 2 and the number of thymine (T) is 1. Accordingly, the methylation degree is calculated to be 2/(2 + 1) = 0.67. Therefore, the DNA methylation status (frequency) in the target site A of Fig. 12B is 0.67 which is a methylation degree derived from 3 cells and can be ascertained as the proportion of cells where DNA methylation has occurred. Meanwhile, the number of cytosine (C) and the number of thymine (T) generated in the target site B is 3 and 0 respectively. Therefore, the methylation degree is calculated to be 3/(3 + 0) = 1. Accordingly, the DNA methylation status (frequency) in the target site A of Fig. 12B is 1 which is a methylation degree derived from 3 cells and can be ascertained as the proportion of cells where DNA methylation has occurred.

Likewise, the methylation status (frequency) of the target site A shown in Fig. 12A can be detected as a methylation degree of 0 derived from one cell, and the methylation status (frequency) of the target site B can be detected as a methylation degree of 1 derived from one cell.

For the amplification of the bisulfite-treated DNA, sometimes multiplex PCR capable of simultaneously amplifying two or more amplification target regions on DNA by the same reaction is used.

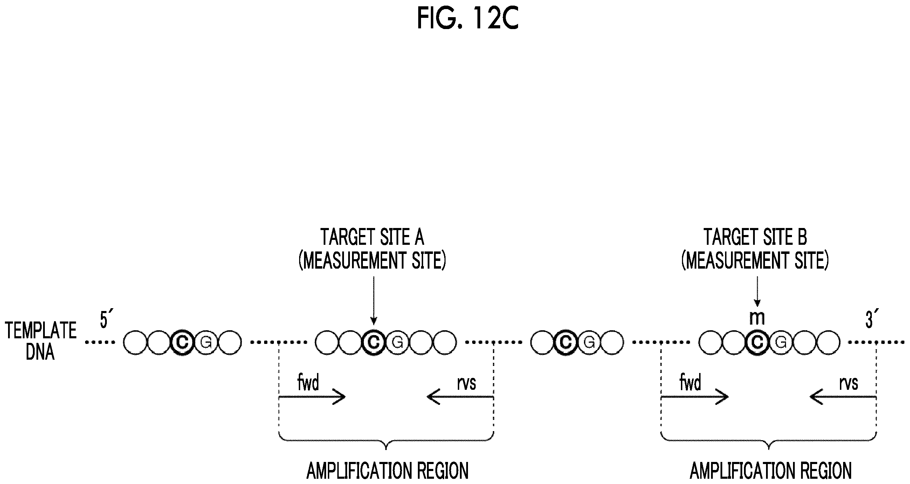

In order to ascertain the DNA methylation status of a predetermined target site or the DNA methylation status (frequency) of a specific target site derived from a plurality of cells by using multiplex PCR, as shown in Fig. 12C (Fig. 12C shows only one strand), it is necessary to use a primer pair (a forward primer and a reverse primer) for amplifying one or more amplification target regions each including one or more target sites. Specifically, as shown in Fig. 12A, it is necessary to use a primer pair for amplifying an amplification target region (amplification region) including the target site A and a primer pair for amplifying an amplification target region (amplification region) including the target site B.

In designing primers for bisulfite-treated DNA, in addition to the conditions considered in the usual primer design (that is, the design of a primer for bisulfite-untreated DNA), the following conditions should also be considered.

First, there is a premise that whether or not DNA methylation will occur is unpredictable unlike in the base sequence. That is, some bases are not sure whether they will be thymine (T) or cytosine (C) after the bisulfite treatment. Therefore, in the primer design for analyzing the DNA methylation status, in order to prevent the amplification efficiency of the primer from changing depending on the methylation status of the periphery of the target site, it is necessary that the primer have no CG sequences in a binding site as far as possible or that the position of CG sequences in the primer be limited to reduce the influence thereof even though the primer includes CG sequences.

In the two strands of DNA, many cytosines (C) on DNA are converted into thymines (T) by the bisulfite treatment. Therefore, in the DNA sequence of each strand, the region configured with three bases other than cytosine (C) increases after the bisulfite treatment. Accordingly, it is also necessary to consider that a primer capable of specifically binding to the region composed of three bases should be designed.

In addition, due to the conversion of many cytosines (C) on DNA into thymines (T), the double-stranded DNA loses the complementarity. Therefore, in a case where both strands of DNA need to be amplified and analyzed, it is necessary to design a primer pair (a forward primer and a reverse primer) for amplifying one or more amplification target regions each including a target site of each strand, that is, two sets of primer pair.

Therefore, compared to designing general primers, designing primers for bisulfite-treated DNA having the aforementioned unique circumstances is more difficult because the design conditions are different.

There are many primer design software, and most of them are for designing general primers, such as Primer-BLAST. Therefore, these software are incapable of setting conditions considering the cytosine that undergoes base conversion by the bisulfite treatment. That is, because the general primer design software does not take into account at all the unique circumstances involved in designing primers for bisulfite-treated DNA as described above, it is impossible to design primers for bisulfite-treated DNA with these software.

Furthermore, in a case where multiplex PCR is used for the amplification of the bisulfite-treated DNA, because a plurality of amplification target regions including each of the target sites relating to the analysis of methylation degree is simultaneously amplified, it is necessary to consider designing a primer suppressing the formation of primer dimers.

Therefore, in a case where a bisulfite reaction or multiplex PCR is used for measuring the methylation degree of DNA of a predetermined site, unfortunately, designing a primer for multiplex PCR used for the analysis (that is, a primer for bisulfite amplicon sequence analysis) is more complicated compared to designing a primer for bisulfite-treated DNA and is time consuming.

As described above, most of the primer design software relates to general primer design software, and few software relates to the design of a primer for bisulfite-treated DNA. In addition, the primer design software for designing a primer for amplifying the bisulfite-treated DNA by multiplex PCR (that is, a primer for bisulfite amplicon sequence analysis) is fewer, and examples of a small number of usable software include the software described in Jennifer Lu and 5 others, "PrimerSuite: A High-Throughput Web-Based Primer Design Program for Multiplex Bisulfite PCR", January 24, 2017, Scientific Reports, Vol. 7, No. 41328.

### SUMMARY OF THE INVENTION

In the bisulfite amplicon sequence analysis, generally, 5 to 1,000 target sites are preset as measurement targets, but it is desirable to output primer sequences at as many target sites as possible. That is, a high primer design success rate (the number of target sites for which the primer can be designed/total number of target sites [%]) is required.

However, generally, it is known that not only the software described in Jennifer Lu and 5 others, "PrimerSuite: A High-Throughput Web-Based Primer Design Program for Multiplex Bisulfite PCR", January 24, 2017, Scientific Reports, Vol. 7, No. 41328, but also the primer design software in the related art for bisulfite-treated DNA has a low primer design success rate. Therefore, there is a demand for a primer design software that can make any improvement of the design success rate of a primer for bisulfite sequence analysis and can more efficiently analyze the DNA methylation status (that is, measure the methylation degree) in a predetermined site.

It is known that in a plant genome, DNA methylation can occur not only in cytosine (C) of a CG sequence but also in cytosine (C) of a CHG sequence and a CHH sequence. However, there is no software for multiplex PCR for these sequences. Therefore, a user who also wants to analyze these sequences should design primers by himself or herself in consideration of all the aforementioned circumstances unique to the design of the primer for bisulfite sequence analysis, which is extremely laborious and time consuming.

The present invention has been made to address the above problems, and an object thereof is to provide a design method, a manufacturing method, a design device, a design program, and a recording medium for a primer for bisulfite amplicon sequence analysis (more specifically, a primer for amplicon methylation sequence analysis) which can improve a design success rate of the primer.

Another object of the present invention is to provide a design method, a manufacturing method, a design device, a design program, and a recording medium for a primer for bisulfite amplicon sequence analysis (more specifically, a primer for amplicon methylation sequence analysis) that is also applicable to cytosine (C) in a CHG sequence and a CHH sequence as cytosine (C) which can be methylated.

The primer design method for amplicon methylation sequence analysis according to an embodiment of the present invention is a method for designing a primer used to simultaneously amplify a plurality of regions each including one or more target sites for measuring a methylation degree by using a bisulfite reaction or an enzyme reaction and multiplex PCR to measure a methylation degree of double-stranded genomic DNA in a predetermined site related to a predetermined biological phenomenon, the method including
a base sequence data acquisition step of acquiring base sequence data of the double-stranded genomic DNA,
a target site information acquisition step of acquiring the one or more target sites and position information thereof,
a base conversion step of converting methylatable "C" into "Y" and converting other "C" into "T" in the base sequence data of the double-stranded genomic DNA,
a complementary strand generation step of generating a complementary strand for each template strand of the double-stranded genomic DNA after base conversion;
a partial sequence cutting step of selecting one target site from the one or more target sites and cutting one or more partial sequences from each strand based on the position information of the selected target site, the one or more partial sequences having a predetermined length from a base sequence positioned on the 5' end side of "Y" formed as a result of conversion of the selected target site or "R" complementary to "Y",
a primer candidate sequence selection step of selecting partial sequences that satisfy predetermined selection conditions as primer candidate sequences from the one or more partial sequences cut out from each strand,
a primer sequence determination step of adopting and determining a forward primer sequence and a reverse primer sequence to amplify a region including the selected target site cut out from each template strand, from the one or more selected primer candidate sequences, and
a repetition step of repeating the partial sequence cutting step, the primer candidate sequence selection step, and the primer sequence determination step until all of the one or more target sites are selected in the partial sequence cutting step,
in which the methylatable "C" is "C" in a CG sequence, and
the predetermined selection conditions include
   (1) a Tm value is within a predetermined range,
   (2) the number of YG sequences or CR sequences included in a partial sequence is equal to or less than a predetermined number, and
   (3) an upper limit of the number of binding sites with a sequence outside a related region on the double-stranded genomic DNA after base conversion is equal to or less than a predetermined number that is equal to or more than 1
[where "C", "G", "Y", and "R" are base codes established by IUPAC, "C" represents cytosine, "G" represents guanine, "Y" represents thymine or cytosine, and "R" represents adenine or guanine].

The methylatable "C" further includes "C" in a CHG sequence, and the predetermined selection conditions can further include (4) the number of YHG sequences or CDR sequences included in the partial sequence is equal to or less than a predetermined number [where "C", "G", "Y", "H", "R", and "D" are base codes established by IUPAC, "C" represents cytosine, "G" represents guanine, "Y" represents thymine or cytosine, "H" represents adenine, cytosine, or thymine, "D" represents thymine, guanine, or adenine, and "R" represents adenine or guanine].

The methylatable "C" further includes "C" in a CHH sequence, and the predetermined selection conditions can further include (5) the number of YHH sequences or DDR sequences included in the partial sequence is equal to or less than a predetermined number [where "Y", "H", "R", and "D" are base codes established by IUPAC, "Y" represents thymine or cytosine, "H" represents adenine, cytosine, or thymine, "D" represents thymine, guanine, or adenine, and "R" represents adenine or guanine].

It is preferable that the predetermined selection conditions further include (6) three bases from the 3' end of the partial sequence are not complementary to three bases from the 3' end of the other partial sequence.

It is preferable that the predetermined selection conditions further include (7) the number of binding sites with the double-stranded genomic DNA before base conversion is equal to or less than a predetermined number.

It is preferable that the predetermined selection conditions further include (8) in a case where the predetermined number of YG sequences or CR sequences included in the partial sequence is set to 1 or more in the condition (2), a range of position of the YG sequences or CR sequences in the partial sequence is also specified, and the number of the YG sequences or CR sequences included in the specified range of position is equal to or less than a predetermined number.

It is preferable that the predetermined selection conditions further include (9) in a case where the predetermined number of YHG sequences or CDR sequences included in the partial sequence is set to 1 or more in the condition (4), a range of position of the YHG sequences or CDR sequences in the partial sequence is also specified, and the number of the YHG sequences or CDR sequences included in the specified range of position is equal to or less than a predetermined number.

It is preferable that the predetermined selection conditions further include (10) in a case where the predetermined number of YHH sequences or DDR sequences included in the partial sequence is set to 1 or more in the condition (5), a range of position of the YHH sequences or DDR sequences in the partial sequence is also specified, and the number of the YHH sequences or DDR sequences included in the specified range of position is equal to or less than a predetermined number.

The primer candidate sequence selection step is
a step of dividing the double-stranded genomic DNA after the base conversion into a first template strand and a second template strand, adopting a complementary strand of the first template strand as a first complementary strand, adopting a complementary strand of the second template strand as a second complementary strand, selecting a partial sequence satisfying predetermined selection conditions as a forward primer candidate sequence of the first template strand among one or more partial sequences cut out from the first template strand, selecting a partial sequence satisfying the predetermined selection conditions as a reverse primer candidate sequence of the first template strand among one or more partial sequences cut out from the first complementary strand, selecting a partial sequence satisfying the predetermined selection conditions as a forward primer candidate sequence of the second template strand among one or more partial sequences cut out from the second template strand, and selecting a partial sequence satisfying the predetermined selection conditions as a reverse primer candidate sequence of the second template strand among one or more partial sequences cut out from the second complementary strand.

The primer sequence determination step is a step of calculating a length of a PCR amplification product predicted to be amplified by PCR for all combinations of the one or more forward primer candidate sequences of the first template strand and the one or more reverse primer candidate sequences of the first template strand selected in the primer candidate sequence selection step, adopting a combination of primer candidate sequences for which the length of the PCR amplification product is calculated to be within a predetermined range as a forward primer sequence and a reverse primer sequence of the first template strand to amplify a region including the target site selected in the partial sequence cutting step, calculating a length of a PCR amplification product predicted to be amplified by PCR for all combinations of the one or more forward primer candidate sequences of the second template strand and the one or more reverse primer candidate sequences of the second template strand selected in the primer candidate sequence selection step, and adopting a combination of primer candidate sequences for which the length of the PCR amplification product is calculated to be within a predetermined range as a forward primer sequence and a reverse primer sequence of the second template strand to amplify a region including the target site selected in the partial sequence cutting step.

After the forward primer sequence and the reverse primer sequence are adopted for all target sites, it is preferable that the primer sequence determination step further calculate local alignment scores for all combinations of the adopted primer sequences and adopts and determines a combination for which the local alignment scores are calculated to be lower than a predetermined threshold value as a primer sequence.

In the condition (3), the upper limit of the number of binding sites with the partial sequence is preferably 1 or 2.

A manufacturing method for a primer for amplicon methylation sequence analysis according to an embodiment of the present invention comprises a primer design step and a synthesis step of synthesizing a primer based on a primer sequence designed in the primer design step, in which the primer design step is performed by the design method for a primer for amplicon methylation sequence analysis described above.

A design device for a primer for amplicon methylation sequence analysis according to an embodiment of the present invention is a device for designing a primer used to simultaneously amplify a plurality of regions each including one or more target sites for measuring a methylation degree by using a bisulfite reaction or an enzyme reaction and multiplex PCR to measure a methylation degree of double-stranded genomic DNA in a predetermined site related to a predetermined biological phenomenon, the design device including
a base sequence data acquisition unit that acquires base sequence data of the double-stranded genomic DNA,
a target site information acquisition unit that acquires the one or more target sites and position information thereof,
a base conversion unit that converts methylatable "C" into "Y" and converting other "C" into "T" in the base sequence data of the double-stranded genomic DNA,
a complementary strand generation unit that generates a complementary strand for each template strand of the double-stranded genomic DNA after base conversion,
a partial sequence cutting unit that selects one target site from the one or more target sites and cuts one or more partial sequences from each strand based on the position information of the selected target site, the one or more partial sequences having a predetermined length from a base sequence positioned on the 5' end side of "Y" formed as a result of conversion of the selected target site or "R" complementary to "Y",
a primer candidate sequence selection unit that selects partial sequences satisfying predetermined selection conditions as primer candidate sequences from the one or more partial sequences cut out from each strand,
a primer sequence determination unit that adopts and determines a forward primer sequence and a reverse primer sequence to amplify a region including the selected target site cut out from each template strand, from the one or more selected primer candidate sequences, and
a control unit that controls the partial sequence cutting unit, the primer candidate sequence selection unit, and the primer sequence determination unit such that each of these units repeat processing thereof until all of the one or more target sites are selected in the partial sequence cutting unit,
in which the methylatable "C" is "C" in a CG sequence, and
the predetermined selection conditions include
   (1) a Tm value is within a predetermined range,
   (2) the number of YG sequences or CR sequences included in a partial sequence is equal to or less than a predetermined number, and
   (3) an upper limit of the number of binding sites with a sequence outside a related region on the double-stranded genomic DNA after base conversion is equal to or less than a predetermined number that is equal to or more than 1
[where "C", "G", "Y", and "R" are base codes established by IUPAC, "C" represents cytosine, "G" represents guanine, "Y" represents thymine or cytosine, and "R" represents adenine or guanine].

The methylatable "C" further includes "C" in a CHG sequence, and the predetermined selection conditions can further include (4) the number of YHG sequences or CDR sequences included in the partial sequence is equal to or less than a predetermined number [where "C", "G", "Y", "H", "R", and "D" are base codes established by IUPAC, "C" represents cytosine, "G" represents guanine, "Y" represents thymine or cytosine, "H" represents adenine, cytosine, or thymine, "D" represents thymine, guanine, or adenine, and "R" represents adenine or guanine].

The methylatable "C" further includes "C" in a CHH sequence, and the predetermined selection conditions can further include (5) the number of YHH sequences or DDR sequences included in the partial sequence is equal to or less than a predetermined number [where "Y", "H", "R", and "D" are base codes established by IUPAC, "Y" represents thymine or cytosine, "H" represents adenine, cytosine, or thymine, "D" represents thymine, guanine, or adenine, and "R" represents adenine or guanine].

It is preferable that the predetermined selection conditions further include (6) three bases from the 3' end of the partial sequence are not complementary to three bases from the 3' end of the other partial sequence.

It is preferable that the predetermined selection conditions further include (7) the number of binding sites with the double-stranded genomic DNA before base conversion is equal to or less than a predetermined number.

It is preferable that the predetermined selection conditions further include (8) in a case where the predetermined number of YG sequences or CR sequences included in the partial sequence is set to 1 or more in the condition (2), a range of position of the YG sequences or CR sequences in the partial sequence is also specified, and the number of the YG sequences or CR sequences included in the specified range of position is equal to or less than a predetermined number.

It is preferable that the predetermined selection conditions further include (9) in a case where the predetermined number of YHG sequences or CDR sequences included in the partial sequence is set to 1 or more in the condition (4), a range of position of the YHG sequences or CDR sequences in the partial sequence is also specified, and the number of the YHG sequences or CDR sequences included in the specified range of position is equal to or less than a predetermined number.

It is preferable that the predetermined selection conditions further include (10) in a case where the predetermined number of YHH sequences or DDR sequences included in the partial sequence is set to 1 or more in the condition (5), a range of position of the YHH sequences or DDR sequences in the partial sequence is also specified, and the number of the YHH sequences or DDR sequences included in the specified range of position is equal to or less than a predetermined number.

The primer candidate sequence selection step is
a step of dividing the double-stranded genomic DNA after the base conversion into a first template strand and a second template strand, adopting a complementary strand of the first template strand as a first complementary strand, adopting a complementary strand of the second template strand as a second complementary strand,
selecting a partial sequence satisfying predetermined selection conditions as a forward primer candidate sequence of the first template strand among one or more partial sequences cut out from the first template strand, selecting a partial sequence satisfying the predetermined selection conditions as a reverse primer candidate sequence of the first template strand among one or more partial sequences cut out from the first complementary strand, selecting a partial sequence satisfying the predetermined selection conditions as a forward primer candidate sequence of the second template strand among one or more partial sequences cut out from the second template strand, and selecting a partial sequence satisfying the predetermined selection conditions as a reverse primer candidate sequence of the second template strand among one or more partial sequences cut out from the second complementary strand.

The primer sequence determination step is a step of calculating a length of a PCR amplification product predicted to be amplified by PCR for all combinations of the one or more forward primer candidate sequences of the first template strand and the one or more reverse primer candidate sequences of the first template strand selected in the primer candidate sequence selection step, adopting a combination of primer candidate sequences for which the length of the PCR amplification product is calculated to be within a predetermined range as a forward primer sequence and a reverse primer sequence of the first template strand to amplify a region including the target site selected in the partial sequence cutting step, calculating a length of a PCR amplification product predicted to be amplified by PCR for all combinations of the one or more forward primer candidate sequences of the second template strand and the one or more reverse primer candidate sequences of the second template strand selected in the primer candidate sequence selection step, and adopting a combination of primer candidate sequences for which the length of the PCR amplification product is calculated to be within a predetermined range as a forward primer sequence and a reverse primer sequence of the second template strand to amplify a region including the target site selected in the partial sequence cutting step.

After the forward primer sequence and the reverse primer sequence are adopted for all target sites, it is preferable that the primer sequence determination step further calculate local alignment scores for all combinations of the adopted primer sequences and adopting and determining a combination for which the local alignment scores are calculated to be lower than a predetermined threshold value as a primer sequence.

In the condition (3), the upper limit of the number of binding sites with the partial sequence is preferably 1 or 2.

The design device for a primer for amplicon methylation sequence analysis further comprises a communication interface, in which the design device is capable of being connected to a server via an external communication network by the communication interface and is capable of operating at least one unit selected from the group consisting of the base sequence data acquisition unit, the target site information acquisition unit, the base conversion unit, the complementary strand generation unit, the partial sequence cutting unit, the primer candidate sequence selection unit, and the primer sequence determination unit by programs in the server.

A design program for a primer for amplicon methylation sequence analysis according to an embodiment of present invention can execute the primer design method on a computer.
A computer-readable recording medium according to an embodiment of the present invention is a medium on which the design program for a primer for amplicon methylation sequence analysis is to be recorded.

According to an embodiment of the present invention, it is possible to improve the design success rate of a primer for bisulfite amplicon sequence analysis (more specifically, a primer for amplicon methylation sequence analysis). In addition, a primer based on the design of the present invention can be obtained. As a result, many target sites can be amplified and measured.

According to the present invention, it is possible to easily and rapidly design a primer for bisulfite amplicon sequence analysis (more specifically, a primer for amplicon methylation sequence analysis) that is also applicable for a CHG sequence and a CHH sequence. In addition, a primer based on the design can be obtained. As a result, the analysis related to these sequences can be performed, which makes it possible to more specifically analyze the DNA methylation status (methylation degree).

### BRIEF DESCRIPTION OF THE DRAWINGS

Fig. 1 is a block diagram conceptually showing an example of the configuration of a primer design device according to a first embodiment of the present invention.
Fig. 2 is a flowchart showing an example of a primer design method according to the first embodiment performed by the primer design device shown in Fig. 1.
Fig. 3A is a schematic view for illustrating a base sequence data acquisition step of the primer design method shown in Fig. 2.
Fig. 3B is a schematic view for illustrating a base conversion step of the primer design method shown in Fig. 2.
Fig. 3C is a schematic view for illustrating a complementary strand generation step of the primer design method shown in Fig. 2.
Fig. 3D is a schematic view for illustrating a partial sequence cutting step of the primer design method shown in Fig. 2.
Fig. 4 is a flowchart showing an example of the operation of a partial sequence cutting unit 28, a primer candidate sequence selection unit 30, and a primer sequence determination unit 32.
Fig. 5A is a view for illustrating the condition (3) "the upper limit of the number of binding sites with a sequence outside the related region on the double-stranded genomic DNA after base conversion is equal to or less than a predetermined number that 1 or more".
Fig. 5B is a view for illustrating the condition (3) "the upper limit of the number of binding sites with a sequence outside the related region on the double-stranded genomic DNA after base conversion is equal to or less than a predetermined number that 1 or more".
Fig. 6 is a flowchart showing an example of a modification example of the condition A of the flowchart shown in Fig. 4.
Fig. 7 is a view for illustrating the condition (6) "three bases from the 3' end of a partial sequence are not complementary to three bases from the 3' end of the other partial sequence".
Fig. 8 is a flowchart showing an example of a modification example of the condition B of the flowchart shown in Fig. 4.
Fig. 9 is a view for illustrating a method of calculating local alignment scores.
Fig. 10 is a block diagram conceptually showing an example of the configuration of a primer design device according to a second embodiment of the present invention.
Fig. 11 is a block diagram conceptually showing an example of the connection between the primer design device according to the second embodiment of the present invention and an external server.
Fig. 12A is a schematic view for illustrating an example of a method of analyzing the methylation status of DNA using a bisulfite reaction.
Fig. 12B is a schematic view for illustrating an example of a method of analyzing methylation status (frequency) of DNA using a bisulfite reaction.
Fig. 12C is a view for illustrating a target site (measurement site) and an amplification target region.

### DESCRIPTION OF THE PREFERRED EMBODIMENTS

Hereinafter, based on public embodiments shown in the accompanying drawings, a design method, a manufacturing method, a design device, a design program and a recording medium for a primer for a bisulfite amplicon sequence (a primer for amplicon methylation sequence analysis) according to embodiments of the present invention will be specifically described.

### (Explanation of terms)

In the present specification, "primer for bisulfite amplicon sequence analysis" means a primer for analysis that is for simultaneously amplifying a plurality of amplification target regions each including a plurality of target sites in bisulfite-treated DNA by multiplex PCR.

"Primer for bisulfite amplicon methylation sequence analysis" means a primer for analysis that is for simultaneously amplifying a plurality of amplification target regions each including a plurality of target sites in bisulfite-treated or enzyme-treated DNA by multiplex PCR.

"Amplification target region" means a region to be amplified by a primer pair.

"Methylation site" means a methylatable site.

"Target site" is a "methylation site" which refers to a site (measurement site) for measuring a methylation degree.

The base sequences such as "GC sequence" and "YG sequence" all mean sequences read from the 5' end side.

A range described using "to" is regarded as including both sides of "to". For example, a range described as "A to B" includes A and B.

### [First embodiment]

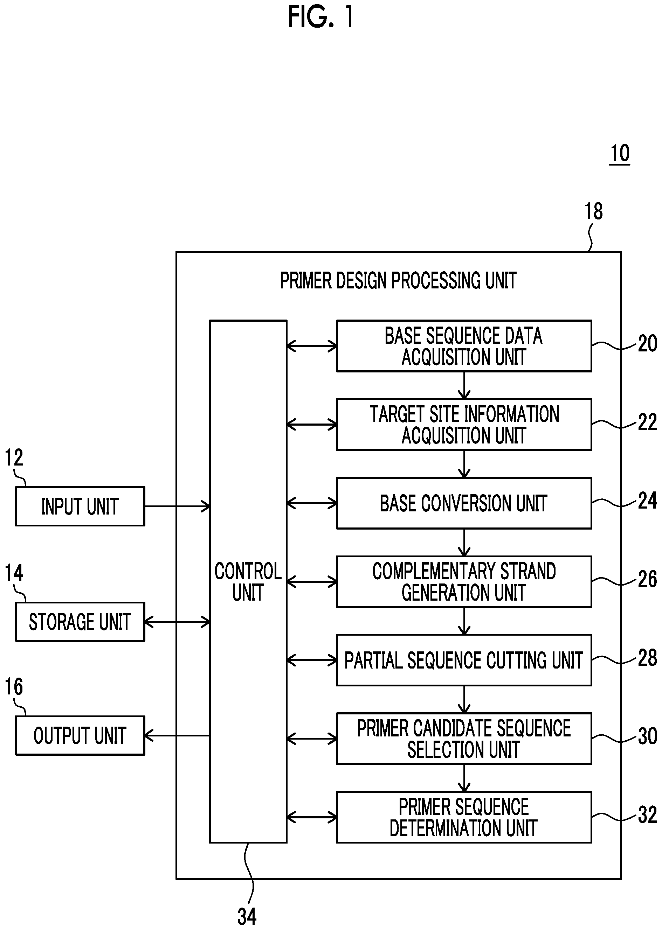

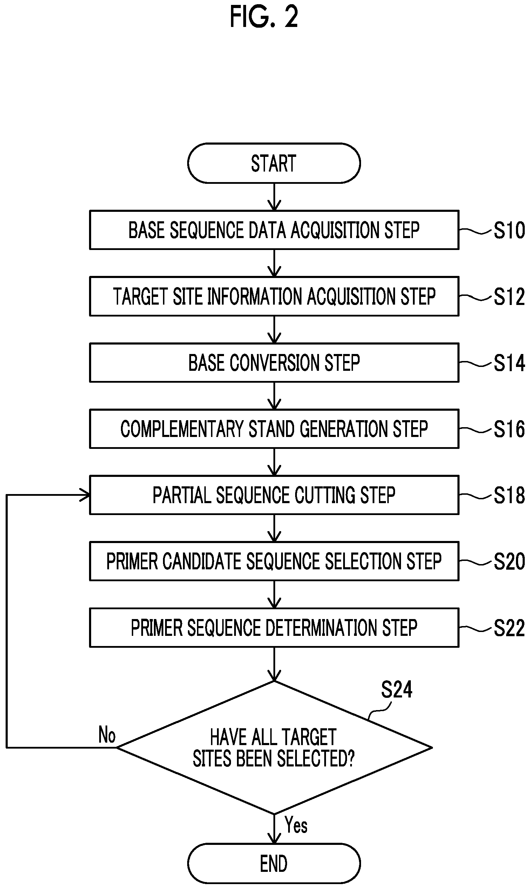

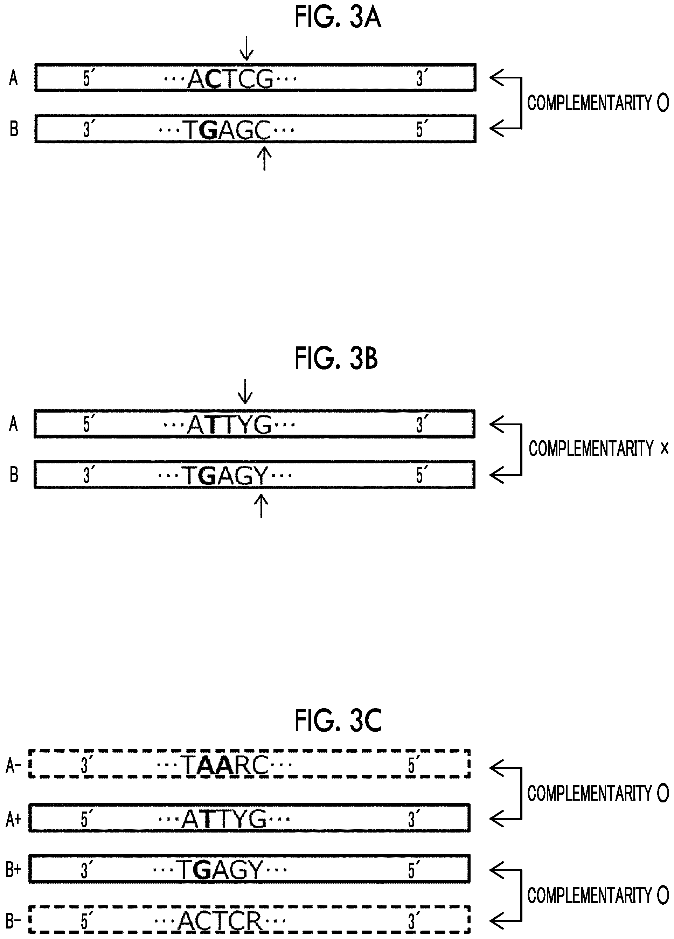

Fig. 1 is a block diagram conceptually showing an example a primer design device according to a first embodiment of the present invention. Fig. 2 is a flowchart showing an example of a primer design method performed by the primer design device shown in Fig. 1. Figs. 3A to 3D are schematic views for illustrating each step of the primer design method.

As shown in Fig. 1, a primer design device 10 comprises an input unit 12, a storage unit 14, an output unit 16, and a primer design processing unit 18. The input unit 12, the storage unit 14, the output unit 16, and the primer design processing unit 18 are connected to each other.

The input unit 12 is a unit that acquires information input by the user, various setting instructions, selection instructions, input instructions, creation instructions, and the like, and is configured with, for example, an input device such as a keyboard and a mouse.

The storage unit 14 stores an operation program of the primer design device, and can also temporarily store information and data necessary for executing primer design processing. As the storage unit 14, for example, it is possible to use recording media such as a hard disc drive (HDD), a solid state drive (SSD), a flexible disc (FD), a magneto-optical (MO) disc, a magnetic tape (MT), a random access memory (RAM), a compact disc (CD), a digital versatile disc (DVD), a secure digital (SD) card, a universal serial bus (USB) memory, and the like.

The output unit 16 is a unit that outputs DNA base sequence information, instructions, design conditions, primer sequence information designed by the primer design processing unit 18, and the like which are input from the input unit 12, and is configured with, for example, display units, such as a liquid crystal display (LCD), organic light-emitting diodes (OLED), flat panel displays, individual displays, and cathode ray tubes (CRT), various types of printers, and the like

The primer design processing unit 18 is a unit that performs a series of processing for primer design.

The primer design processing unit 18 comprises a base sequence data acquisition unit 20, a target site information acquisition unit 22, a base conversion unit 24, a complementary strand generation unit 26, a partial sequence cutting unit 28, a primer candidate sequence selection unit 30, a primer sequence determination unit 32, and a control unit 34.

The primer design processing unit 18 can be configured with a processor including a central processing unit (CPU) or the like, a computer, and the like.

As shown in Fig. 2, a primer design method 10 includes a base sequence data acquisition step S10, a target site information acquisition step S12, a base conversion step S14, a complementary strand generation step S16, a partial sequence cutting step S18, a primer candidate sequence selection step S20, a primer sequence determination step S22, and a repetition step of repeating the partial sequence cutting step S18, the primer candidate sequence selection step S20, and the primer sequence determination step S22 until all target sites are detected by a determination step S24.

### (Base sequence data acquisition unit)

The base sequence data acquisition unit 20 shown in Fig. 1 is a unit that performs the base sequence data acquisition step S10 shown in Fig. 2, and acquires the data of the double-stranded DNA sequence (reference sequence) of the genome of biological species, for which a primer is to be designed, via the input unit 12. In a case where the data of the reference sequence is stored in the storage unit 14 in advance, the data may be acquired from the storage unit 14.

It is preferable that the data of the double-stranded genomic DNA sequence to be acquired be the data of the complete sequence of the genome of the biological species for which a primer is to be designed.

In order to explain the primer design method of the present embodiment, the double-stranded DNA of the double-stranded DNA sequence data acquired in this step will be called template DNA which will be referred to as a strand A and a strand B, respectively (see Fig. 3A).

The base sequence data acquisition unit 20 is configured with a computer and functions to acquire the data of the double-stranded DNA sequence of the genome described above.

### (Target site information acquisition unit)

The target site information acquisition unit 22 shown in Fig. 1 is a unit that performs the target site information acquisition step S12 shown in Fig. 2, and can acquire one or more target sites included in the double-stranded genomic DNA acquired by the base sequence data acquisition unit 20 and the position information of the target sites via the input unit 12. In a case where the target sites and the position information thereof are stored in the storage unit 14 in advance, the target sites and the position information thereof may be acquired from the storage unit 14.

"Target site" is a site related to a predetermined biological phenomenon, is cytosine (C) of a CG sequence which is methylatable cytosine (C), and is a site for measuring a methylation degree.

The number of target sites to be selected is not particularly limited. From the viewpoint of markedly obtaining the desired effect of the present invention, it is preferable to select 5 to 1,000 sites.

The position of each target site can be indicated by a chromosome, a genomic coordinate, or the like.

The target site information acquisition unit 22 is configured with a computer and functions to acquire one or more target sites included in the aforementioned double-stranded genomic DNA and position information thereof.

### (Base conversion unit)

The base conversion unit 24 is a unit that performs the base conversion step S14 shown in Fig. 2. As shown in Figs. 3A and 3B, the base conversion unit 24 converts cytosine (C) of a CG sequence on the template DNA acquired from the base sequence data acquisition unit 20 into "Y" (see the bases indicated by the arrows in Figs. 3A and 3B) and converts cytosine (C) of other sequences into thymine (T). Cytosine (C) in a CG sequence of DNA is likely to be methylated or unmethylated. Therefore, cytosine (C) is converted into "Y" having both the possibility of being converted into thymine (T) and the possibility of remaining as cytosine (C).

Note that this conversion processing is computer simulation that reproduces the generation of DNA amplified by PCR after a bisulfite treatment.

The base conversion unit 24 is configured with a computer and functions to convert cytosine (C) of the CG sequence on the aforementioned template DNA into "Y" and cytosine (C) of other sequences into thymine (T).

As described above, due to the bisulfite treatment, the DNA double strands lose the complementarity thereof. This is because the bisulfite treatment induces the conversion of the cytosine (C) of the CG base pair having complementarity into the thymine (T), which removes the complementarity of the base pair (see the bolded bases in Figs. 3A and 3B). With one set of primers, it is impossible to equally amplify both strands of the amplification target region on the bisulfite-treated DNA having lost the complementarity in this way. Therefore, in a case where the methylation status of double-stranded DNA is to be analyzed, a primer pair (a forward primer and a reverse primer) for amplifying an amplification target region including each target site of each strand needs to be prepared for each target site. That is, it is necessary to design a primer pair related to the amplification target region including the target site of the strand A after base conversion in Fig. 3B and a primer pair related to the amplification target region including the target site of the strand B after base conversion, respectively.

However, as will be explained in Modification Example 7 that will be described later, in a case where the user wants to analyze only the strand A or strand B, or in a case where it will be fine if either the strand A or the strand B can be analyzed, it is not necessary to design two sets of primer pair.

### (Complementary strand generation unit)

The complementary strand generation unit 26 is a unit that performs the complementary strand generation step S16 shown in Fig. 2, and generates a complementary strand for each of two DNA strands after the base conversion processing.

In order to illustrate the primer design method of the present embodiment, the strand A after base conversion and the strand B after base conversion will be called a first template strand (strand A+) and a second template strand (strand B+) respectively, and a complementary strand of the first template strand and a complementary strand of the second template strand will be called a first complementary strand (strand A-) and a second complementary strand (strand B-) respectively (see Fig. 3C).

As shown in Fig. 3C, a sequence complementary to the base sequence of the strand A+ is generated to prepare a complementary strand A-, and a sequence complementary to the base sequence of the strand B+ is generated to prepare a complementary strand B-. The base complementary to "Y" is denoted by "R" having both the possibility of being adenine (A) and the possibility of being guanine (G).

The complementary strand generation unit 26 is configured with a computer and functions to generate the aforementioned complementary strand for each of the two strands of DNA after base conversion processing.

As a result, the first template strand (strand A+) is configured with three bases of thymine (T), adenine (A), and guanine (G) excluding "Y" (that is, a methylation site), the first complementary strand (strand A-) is configured with three bases of thymine (T), adenine (A), and cytosine (C) excluding "R" (a methylation site), and the first template strand (strand A+) and the first complementary strand (strand A-) can have complementarity.

Likewise, the second template strand (strand B+) is configured with three bases of thymine (T), adenine (A), and guanine (G) excluding "Y" (a methylation site), the second complementary strand (strand B-) is configured with three bases of thymine (T), adenine (A), and cytosine (C) excluding "R" (a methylation site), and the second template strand (strand B+) and the second complementary strand (strand B-) can have complementarity.

### (Partial sequence cutting unit)

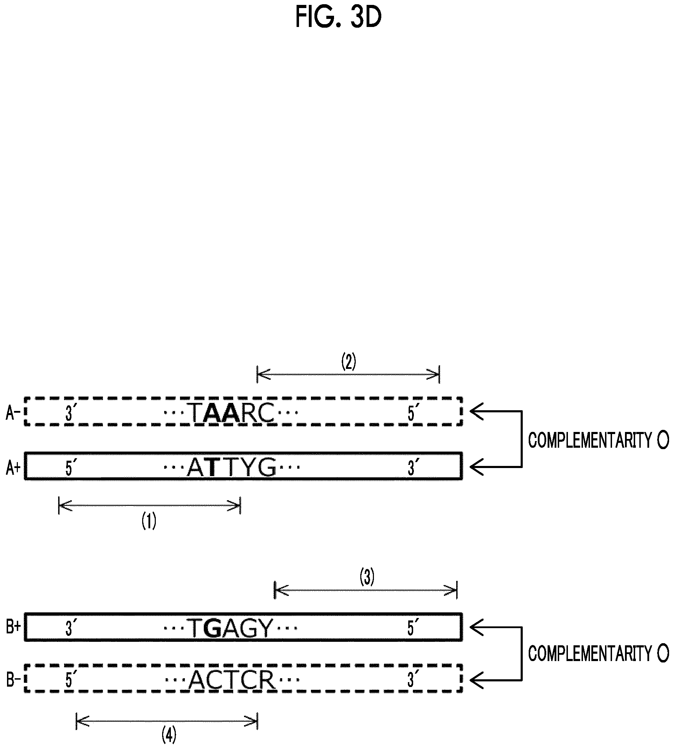

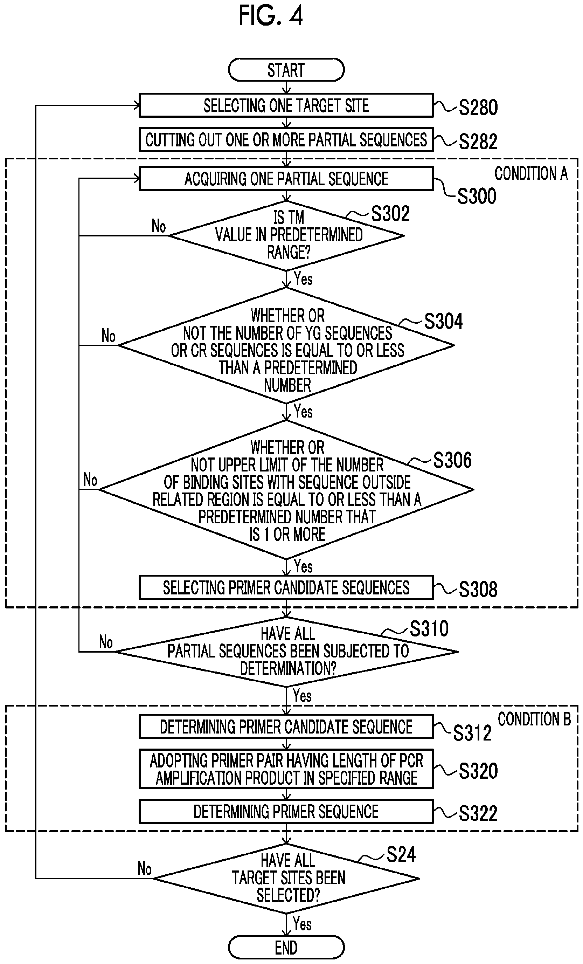

The partial sequence cutting unit 28 is a unit that performs the partial sequence cutting step S18 shown in Fig. 2. As shown in the flowchart of Fig. 4, the partial sequence cutting unit 28 selects one target site from one or more target sites acquired by the target site information acquisition unit 22 (step S280), detects "Y" of the selected target site or "R" (that is, a base which is in a methylation site in the target site) complementary to "Y" from the DNA sequence of each strand based on the position information of the selected target site, and cuts partial sequences as much as possible from the partial sequences having a predetermined length from the base sequences ((1) to (4) in Fig. 3D) positioned on the 5' end side of the detected "Y" or "R" (step S282) to obtain one or more partial sequences.

Fig. 4 is a flowchart showing an example of the operation of a partial sequence cutting unit 28, a primer candidate sequence selection unit 30, and a primer sequence determination unit 32.

The partial sequence cutting unit 28 is configured with a computer and functions to cut partial sequences as much as possible from partial sequences having a predetermined length from "Y" of the selected target site or "R" complementary to "Y"from the DNA sequence of each strand based on the position information of the selected target site described above to obtain one or more partial sequences.

The length of one or more partial sequences to be cut out is not particularly limited. From the viewpoint of processing efficiency and markedly obtaining the desired effect of the present invention, it is preferable that the length of one or more partial sequences to be cut out be equal to maximum length of PCR amplification product that the user desires - minimum length of primer - length (one base) of target site.

The length of the PCR amplification product is not particularly limited as long as it is in a known range, that is, 70 to several kilo base pairs. It is preferable to consider a PCR success rate, the sequencing ability of a DNA sequencer, and the like.

The length of the primer is not particularly limited as long as it is in a known range, that is, 15 to 45 bases. It is preferable to consider the specificity of the primer and the primer dimer forming properties.

For example, in a case where the maximum length of the PCR product set by the user is 300 bases and the minimum length of the primer is 20 bases, a predetermined length x to be cut out is calculated by x = 300 - 20 - 1 (length of the target site), which is equal to 279. Therefore, first, 279 bases on the 5' end side of each target site are cut out. As shown in Fig. 3D, a target site of each strand, that is, 279 bases on the 5' end side of "Y" of the strand A+, "R" of the strand A-, "Y" of the strand B+, and "R" of the strand B- ((1) to (4) in Fig. 3D) are cut out from each strand.

Subsequently, by cutting partial sequences from the 279 bases in the length of the primer (equal to or less than a predetermined length consisting of 20 or more bases) as much as possible, it is possible to obtain one or more partial sequences.

The numerical value or numerical range of the length of the PCR amplification product and the length of the primer are set by the user via the input unit 12. In a case where these conditions are stored in the storage unit 14 in advance, these conditions can be set by being acquired from the storage unit 14.

### (Primer candidate sequence selection unit)

The primer candidate sequence selection unit 30 is a unit that performs the primer candidate sequence selection step S20 shown in Fig. 2, and selects partial sequences satisfying all the predetermined selection conditions (1) to (3) as primer candidate sequences from one or more partial sequences of each strand cut out by the partial sequence cutting unit 28.

Specifically, a partial sequence that satisfies the predetermined selection conditions is selected as a forward primer candidate sequence of the first template strand (strand A+) among one or more partial sequences cut out from the first template strand (strand A+) (that is, one or more partial sequences cut out from (1) in Fig. 3D), a partial sequence that satisfies the predetermined selection conditions is selected as a reverse primer candidate sequence of the first template strand (strand A+) among one or more partial sequences cut out from the first complementary strand (strand A-) (that is, one or more partial sequences cut out from (2) in Fig. 3D), a partial sequence that satisfies the predetermined selection conditions is selected as a forward primer candidate sequence of the second template strand (strand B+) among one or more partial sequences cut out from the second template strand (strand B+) (that is, one or more partial sequences cut out from (3) in Fig. 3D), and a partial sequence that satisfies the predetermined selection conditions is selected as a reverse primer candidate sequence of the second template strand (strand B+) among one or more partial sequences cut out from the second complementary strand (strand B-) (that is, one or more partial sequences cut out from (4) in Fig. 3D).

The primer candidate sequence selection unit 30 is configured with a computer and functions to select partial sequences that satisfy all the predetermined selection conditions (1) to (3) as primer candidate sequences from one or more partial sequences of each strand described above.

"Predetermined selection conditions" of the primer candidate sequences are conditions (1) to (3) described below. The user can preset the numerical value and numerical range of the predetermined selection conditions via the input unit 12.
(1) a Tm value is within a predetermined range.
(2) The number of YG sequences or CR sequences included in a partial sequence is equal to or less than a predetermined number.
(3) An upper limit of the number of binding sites with a base sequence outside a related region on the template strand DNA (double-stranded genomic DNA) after base conversion is equal to or less than a predetermined number that is equal to or more than 1.

The range of "Tm value" related to the condition (1) is not particularly limited as long as it is in a known numerical range, that is, 45°C to 70°C. It is preferable to consider the thermal cycle conditions of PCR, the ease of PCR amplification (the temperature range in which amplification can easily proceed by the PCR enzyme used), and the specificity of PCR amplification. The Tm value can be calculated by, for example, the nearest neighbor base pair method.

The number of "YG sequences or CR sequences included in a partial sequence" related to the condition (2) is not particularly limited. From the viewpoint of markedly obtaining the desired effect of the present invention, the number of YG sequences or CR sequences is preferably 2 or less, more preferably 1 or less, and particularly preferably 0.

In a case where the above condition is satisfied, the influence of the binding of the primer to cytosine (C) of the CG sequence in the primer binding site can be reduced.

"Sequence outside the related region on the template strand DNA (double-stranded genomic DNA) after base conversion" related to the condition (3) described above refers to the base sequence excluding the sequence at the position on the template strand DNA after base conversion, the position corresponding to the partial sequence, and a base sequence complementary to the sequence (the template strand DNA sequence after base conversion) excluding the partial sequence.

"Upper limit of the number of binding sites with the sequence outside the related region on the template strand DNA after base conversion" is not particularly limited. From the viewpoint of markedly obtaining the desired effect of the present invention, the upper limit of the number of such binding sites is preferably 5 or less, and particularly preferably 2 or less.

In a case where the above condition is satisfied, the influence of binding of the primer to the outside of the related region on the bisulfite-treated DNA can be reduced.

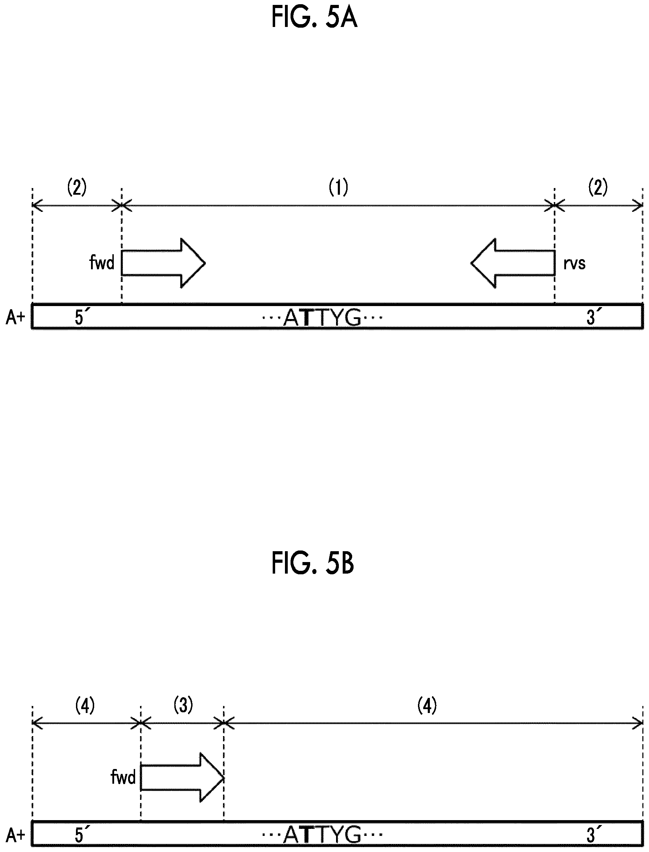

In a case where the number of heating cycles in PCR is set to n, and a primer pair (a forward primer and a reverse primer) binds to DNA as shown in Fig. 5A, PCR amplification products are generated in the order of 2". In contrast, in a case where either the forward primer or the reverse primer binds to DNA as shown in Fig. 5B, PCR amplification products are generated in the order of 2ⁿ (Fig. 5B shows a case where the forward primer binds to DNA).

Therefore, in a case where PCR is performed using a general number of heating cycles (n is about 20 to 40), and a primer pair binds to a DNA sequence outside the amplification target region, unfortunately, non-specific products are generated in large amounts. However, in a case where either the forward primer or the reverse primer binds to the DNA sequence outside the related region, the amounts of generated non-specific products are not that large, which does not cause a special problem. Accordingly, in the related art, the problem of non-specific products being generated in a case where either the forward primer or the reverse primer binds to the DNA sequence outside the related region has not been especially considered. In Fig. 5A, (1) is the DNA sequence of the amplification target region, and (2) is the DNA sequence outside the amplification target region. Furthermore, in Fig. 5B, (3) is the DNA sequence of the related region of a partial sequence, and (4) is the DNA sequence outside the related region.

As a result of repeating intensive studies, the inventors of the present invention have found that it is possible to increase the primer design success rate by performing determination under conditions created by adding the condition (3), which allows each primer to bind to DNA outside the target region within a predetermined range, to the condition of the related art in which determination is performed in designing a primer.

The processing of selecting partial sequences satisfying predetermined selection conditions as primer candidate sequences among one or more partial sequences cut out from each strand will be described using the flowchart in Fig. 4.

First, the primer candidate sequence selection unit 30 acquires one partial sequence from one or more partial sequences cut out from the first template strand (strand A+) (step S300) and determines whether or not the Tm value of the partial sequence is within a predetermined range (step S302).

In a case where the Tm value is not within a predetermined range, the primer candidate sequence selection unit 30 acquires another partial sequence (step S300). In a case where the Tm value is within a predetermined range, the primer candidate sequence selection unit 30 determines whether or not the number of YG sequences or CR sequences included in the partial sequence is equal to or less than a predetermined number. (Step S304).

In a case where the number of YG sequences or CR sequences included in the partial sequence is not equal to or less than a predetermined number, the primer candidate sequence selection unit 30 acquires another partial sequence (step S300). In a case where the number of YG sequences or CR sequences included in the partial sequence is equal to or less than a predetermined number, the primer candidate sequence selection unit 30 determines whether or not the upper limit of the number of binding sites with the base sequence outside the related region on the template strand DNA after base conversion is equal to or less than a predetermined number which is 1 or more (step S306).

In a case where the upper limit of the number of binding sites between the base sequence outside the related region on the template strand DNA after base conversion and the partial sequence is not equal to or less than "a predetermined number which is 1 or more", the primer candidate sequence selection unit 30 acquires another partial sequence (step S300). In a case where the upper limit of the number of binding sites with the sequence outside the related region on the template strand DNA after base conversion is equal to or less than a predetermined number which is 1 or more, the primer candidate sequence selection unit 30 selects the partial sequence as a primer candidate sequence (step S308) and determines whether or not all the partial sequences cut out from the first template strand (strand A+) have been subjected to determination (step S310).

In a case where not all the partial sequences cut out from the first template strand (strand A+) have been subjected to determination, the primer candidate sequence selection unit 30 acquires another partial sequence (step S300). In a case where all the partial sequences have been subjected to determination, the primer candidate sequence selection unit 30 determines one or more selected primer candidate sequences as forward primer candidate sequences of the first template strand (strand A+) (step S312).

One or more partial sequences cut out from the first complementary strand (strand A-), one or more partial sequences cut out from the second template strand (strand B+), and one or more partial sequences cut out from the second complementary strand (strand B-) are subjected to the same determination (steps S300 to S310), and reverse primer candidate sequences of the first template strand (strand A+), forward primer candidate sequences of the second template strand (strand B+), and reverse primer candidate sequences of the second template strand (strand B+) are determined (step S312).

### (Primer sequence determination unit)

The primer sequence determination unit 32 is a unit that performs the primer sequence determination step S22 shown in Fig. 2. From one or more primer candidate sequences determined by the primer candidate sequence selection unit 30, that is, from one or more forward primer candidate sequences of the first template strand (strand A+), one or more reverse primer candidate sequences of the first template strand (strand A+), one or more forward primer candidate sequences of the second template strand (strand B+), and one or more reverse primer candidate sequences of the second template strand (strand B+), a forward primer sequence and a reverse primer sequence to amplify the region including the target site selected by the partial sequence cutting unit 28 are determined and adopted by the primer sequence determination unit 32.

The primer sequence determination unit 32 is configured with a computer and functions to adopt and determine a forward primer sequence and a reverse primer sequence from one or more primer candidate sequences described above.

Specifically, first, the primer sequence determination unit 32 acquires all primer pairs (combinations of a forward primer and a reverse primer) producible from one or more forward primer candidate sequences of the first template strand and one or more reverse primer candidate sequences of the first template strand, and calculates the lengths of PCR amplification products predicted to be amplified by PCR for each primer pair. Then, the primer sequence determination unit 32 determines whether or not the calculated length of each PCR amplification product is within a predetermined numerical range. In a case where the calculated length of the PCR amplification product is within a predetermined numerical range, the primer sequence determination unit 32 adopts the primer pair for which the length of the PCR amplification product is calculated (that is, a combination of the forward primer candidate sequence of the first template strand and the reverse primer candidate sequence of the first template strand) as a forward primer sequence and a reverse primer sequence of the first template strand to amplify the region including the target site selected by the partial sequence cutting unit 28 (partial sequence cutting step S18) (step S320) and determines these sequences as a primer sequence (step S322).

Likewise, first, the primer sequence determination unit 32 acquires all primer pairs (combinations of a forward primer and a reverse primer) producible from one or more forward primer candidate sequences of the first template strand and one or more reverse primer candidate sequences of the second template strand, and calculates the lengths of PCR amplification products predicted to be amplified by PCR for each primer pair.

Then, the primer sequence determination unit 32 determines whether or not the calculated length of each PCR amplification product is within a predetermined numerical range (step S320). In a case where the calculated length of the PCR amplification product is within a predetermined numerical range, the primer sequence determination unit 32 adopts the primer pair for which the length of the PCR amplification product is calculated (that is, a combination of the forward primer candidate sequence of the second template strand and the reverse primer candidate sequence of the second template strand) as a forward primer sequence and a reverse primer sequence of the second template strand to amplify the region including the target site selected by the partial sequence cutting unit 28 (partial sequence cutting step S18) and determines these sequences as a primer sequence (step S322).

"Predetermined numerical range" for determining the calculated length of the PCR amplification product is a range including the length of the PCR amplification product that the user desires. As described above, the predetermined numerical range is not particularly limited as long as it is a known range, that is, 70 to several kilo base pairs. It is preferable to consider a PCR success rate, the sequencing ability of a DNA sequencer, and the like.

Once the determination of whether or not the length of a PCR amplification product is within a predetermined range is completed for all primer pairs, whether or not all target sites have been selected in the partial sequence cutting unit 28 (partial sequence cutting step S18) is determined (S24).

In a case where not all the target sites have been selected, the processing returns to the partial sequence cutting step S18 to select other target sites (step S280). In a case where all the target sites have been selected, the processing ends.

### (Control unit)

The control unit 34 is a unit that is connected not only to the portions in the primer design processing unit 18 but also to the input unit 12, the storage unit 14, and the output unit 16 directly or indirectly, controls each unit of the primer design device 10 based on the user's instruction from the input unit 12 or based on a predetermined operation program stored in the storage unit 14, and designs a primer. The control unit 34 is configured with, for example, a central processing unit (CPU) of a computer or the like.

The control unit 34 controls the primer candidate sequence selection unit 30, such that the determination operation (steps S300 to S308) is repeated until the determination of whether or not all the partial sequences satisfy a predetermined selection standard is completed in the primer candidate sequence selection unit 30 (step S310).

The control unit 34 controls the primer sequence determination unit 32, such that the determination operation (step S320) is repeated until the determination of whether or not the length of a PCR amplification product is within a predetermined range is completed for all the produced primer pairs in the primer sequence determination unit 32.

The control unit 34 controls the partial sequence cutting unit 28, the primer candidate sequence selection unit 30, and the primer sequence determination unit 32, such that the repetition step of repeating the partial sequence cutting step (steps S18 and S280 to S282), the primer candidate sequence selection step (step S20 and S300 to S312), and the primer sequence determination step (steps S22 and S320 to S322) is carried out until all the target sites acquired by the target site information acquisition unit 22 are detected in the partial sequence cutting unit 28 (step S24).

With the primer design device 10 according to the first embodiment of the present invention, it is possible to design a primer for amplicon methylation sequence analysis with an excellent design success rate. In addition, a primer based on the design can be obtained. As a result, it is possible to design a primer for more target sites and measure the methylation degree.

### [Modification Example 1]

Next, a primer design device according to Modification Example 1 of the first embodiment of the present invention will be described. Regarding the primer design device according to Modification Example 1, the same processing as that of the first embodiment will not be described.

In the first embodiment, the methylatable cytosine (C) is limited to cytosines (C) in the CG sequence, and cytosine (C) picked up from such cytosines (C) is adopted as a target site. However, the methylatable cytosine (C) is not limited thereto and may include cytosines (C) in a CHG sequence, and cytosine (C) picked up from such cytosines may be adopted as a target site.

In Modification Example 1, the target site information acquisition unit 22 additionally acquires one or more target sites included in the double-stranded genomic DNA acquired by the base sequence data acquisition unit 20 and the position information of the target sites via the input unit 12.

The base conversion unit 24 also converts cytosine (C) of a CHG sequence on the template DNA acquired from the base sequence data acquisition unit 20 into "Y", and converts cytosine (C) of other sequences (that is, sequences other than a CG sequence and a CHG sequence) into thymine (T).

The primer candidate sequence selection unit 30 additionally selects partial sequences satisfying all the predetermined selection conditions (1) to (4) including the following condition (4) as primer candidate sequences, among one or more partial sequences of each strand cut out by the partial sequence cutting unit 28.

(4) The number of YHG sequences or CDR sequences included in a partial sequence is equal to or less than a predetermined number.

The number of "YHG sequences or CDR sequences included in a partial sequence" related to the condition (4) is not particularly limited. From the viewpoint of markedly obtaining the desired effect of the present invention, the number of YHG sequences or CDR sequences is preferably 2 or less, more preferably 1 or less, and particularly preferably 0.

In a case where the above condition is satisfied, the influence of the binding of the primer to cytosine (C) of the CHG sequence in the primer binding site can be reduced.

With the primer design device of Modification Example 1 according to the first embodiment of the present invention, it is possible to easily and rapidly design a primer for amplicon methylation sequence analysis that is also applicable to a CHG sequence. In addition, a primer based on the design can be obtained. As a result, the analysis related to these sequences can be performed, which makes it possible to more specifically analyze the DNA methylation status (methylation degree).

### [Modification Example 2]

Next, a primer design device according to Modification Example 2 of the first embodiment of the present invention will be described. Regarding the primer design device according to Modification Example 2, the same processing as that of the first embodiment will not be described.

In the first embodiment, the methylatable cytosine (C) is limited to cytosines (C) in the CG sequence, and cytosine (C) picked up from such cytosines (C) is adopted as a target site. However, the methylatable cytosine (C) is not limited thereto and may include cytosines (C) in a CHH sequence, and cytosine (C) picked up from such cytosines may be adopted as a target site.

In Modification Example 2, the target site information acquisition unit 22 additionally acquires one or more target sites included in the double-stranded genomic DNA acquired by the base sequence data acquisition unit 20 and the position information of the target sites via the input unit 12.

The base conversion unit 24 also converts cytosine (C) of a CHH sequence on the template DNA acquired from the base sequence data acquisition unit 20 into "Y", and converts cytosine (C) of other sequences (that is, sequences other than a CG sequence and a CHH sequence) into thymine (T).

The primer candidate sequence selection unit 30 additionally selects partial sequences satisfying all the predetermined selection conditions (1) to (3) and (5) including the following condition (5) as primer candidate sequences, among one or more partial sequences of each strand cut out by the partial sequence cutting unit 28.

(5) The number of YHH sequences or DDR sequences included in a partial sequence is equal to or less than a predetermined number.

The number of "YHH sequences or DDR sequences included in a partial sequence" related to the condition (5) is not particularly limited. From the viewpoint of markedly obtaining the desired effect of the present invention, the number of YHH sequences or DDR sequences is preferably 2 or less, more preferably 1 or less, and particularly preferably 0.

In a case where the above condition is satisfied, the influence of the binding of the primer to cytosine (C) of the CHH sequence in the primer binding site can be reduced.

With the primer design device of Modification Example 2 according to the first embodiment of the present invention, it is possible to easily and rapidly design a primer for amplicon methylation sequence analysis that is also applicable to a CHH sequence. In addition, a primer based on the design can be obtained. As a result, the analysis related to these sequences can be performed, which makes it possible to more specifically analyze the DNA methylation status (methylation degree).

Modification Example 2 can be combined with Modification Example 1 described above. That is, the methylatable cytosine (C) may include both the cytosine (C) in a CHG sequence and the cytosine (C) in a CHH sequence, and cytosine (C) picked up from the above cytosines may be adopted as a target site.

In this case, the primer candidate sequence selection unit 30 additionally selects partial sequences satisfying all the selection conditions (1) to (5) as primer candidate sequences, among one or more partial sequences cut out by the partial sequence cutting unit 28.

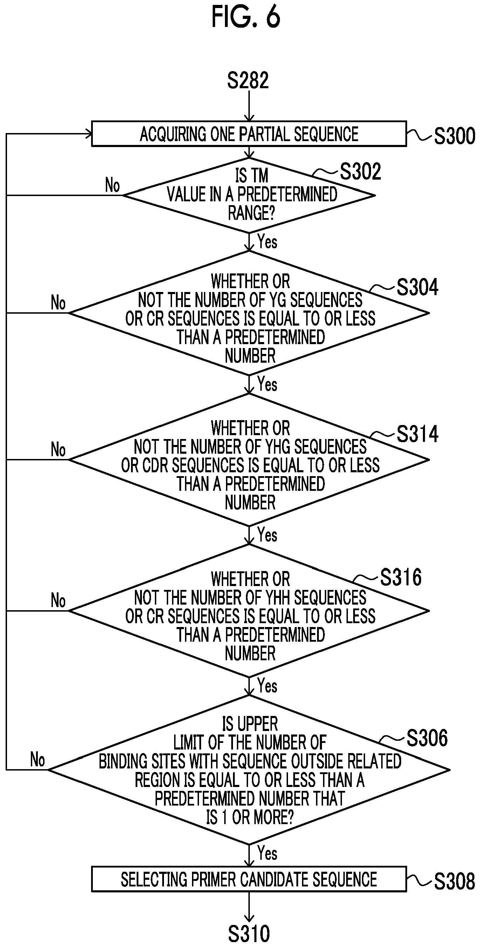

The processing of selecting partial sequences satisfying the selection conditions (1) to (5) as primer candidate sequences among one or more partial sequences cut out from each strand will be described using the flowchart in Fig. 6. Fig. 6 is a flowchart showing a modification example of the condition A of Fig. 4, and the same processing as that of the first embodiment will not be described.

First, the primer candidate sequence selection unit 30 acquires one partial sequence from one or more partial sequences cut out from the first template strand (strand A+) (step S300) and determines whether or not the Tm value of the partial sequence is within a predetermined range (step S302).

In a case where the Tm value is not within a predetermined range, the primer candidate sequence selection unit 30 acquires another partial sequence (step S300). In a case where the Tm value is within a predetermined range, the primer candidate sequence selection unit 30 determines whether or not the number of YG sequences or CR sequences included in the partial sequence is equal to or less than a predetermined number. (Step S304).

In a case where the number of YG sequences or CR sequences included in the partial sequence is not equal to or less than a predetermined number, the primer candidate sequence selection unit 30 acquires another partial sequence (step S300). In a case where the number of YG sequences or CR sequences included in the partial sequence is equal to or less than a predetermined number, the primer candidate sequence selection unit 30 determines whether or not the number of YHG sequences or CHR sequences included in the partial sequence is equal to or less than a predetermined number (step S314).

In a case where the number of YHG sequences or CHR sequences included in the partial sequence is not equal to or less than a predetermined number, the primer candidate sequence selection unit 30 acquires another partial sequence (step S300). In a case where the number of YHG sequences or CHR sequences included in the partial sequence is equal to or less than a predetermined number, the primer candidate sequence selection unit 30 determines whether or not the number of YHH sequences or DDR sequences included in the partial sequence is equal to or less than a predetermined number (step S316).

In a case where the number of YHH sequences or DDR sequences included in the partial sequence is not equal to or less than a predetermined number, the primer candidate sequence selection unit 30 acquires another partial sequence (step S300). In a case where the number of YHH sequences or DDR sequences included in the partial sequence is equal to or less than a predetermined number, the primer candidate sequence selection unit 30 determines whether or not the upper limit of the number of binding sites with the sequence outside the related region on the template strand DNA after base conversion is equal to or less than a predetermined number which is 1 or more (step S306).

In a case where the upper limit of the number of binding sites with the sequence outside the related region on the template strand DNA having undergone base conversion is not equal to or less than a predetermined number which is 1 or more, the primer candidate sequence selection unit 30 acquires another partial sequence (step S300). In a case where the upper limit of the number of binding sites with the sequence outside the related region on the template strand DNA having undergone base conversion is equal to or less than a predetermined number which is 1 or more, the primer candidate sequence selection unit 30 selects the partial sequence as a primer candidate sequence (step S308) and determines whether or not all the partial sequences cut out from the first template strand (strand A+) have been subjected to determination (step S310).

In a case where not all the partial sequences cut out from the first template strand (strand A+) have been subjected to determination, the primer candidate sequence selection unit 30 acquires another partial sequence (step S300). In a case where all the partial sequences have been subjected to determination, the primer candidate sequence selection unit 30 determines one or more selected primer candidate sequences as forward primer candidate sequences of the first template strand (strand A+) (step S312).

In a case where the above condition is satisfied, the influence of the binding of the primer to cytosine (C) of the CHG sequence in the primer binding site and the influence of the binding of the primer to cytosine (C) of the CHH sequence can be reduced.

### [Modification Example 3]

Next, a primer design device according to Modification Example 3 of the first embodiment of the present invention will be described. Regarding the primer design device according to Modification Example 3, the same processing as that of the first embodiment will not be described.

In the present embodiment, in the primer candidate sequence selection unit 30, the conditions (1) to (3) are set as predetermined selection conditions used for selecting primer candidate sequences. However, it is preferable that the predetermined selection conditions further include the following condition (6).

(6) Three bases from the 3' end of a partial sequence are not complementary to three bases from the 3' end of the other partial sequence.

No complementarity between three bases from the 3' end of a partial sequence means that three bases from the 3' end of the primer have no complementarity in all nC₂ combinations of primers obtained by combining two primers selected from n pieces of primers.

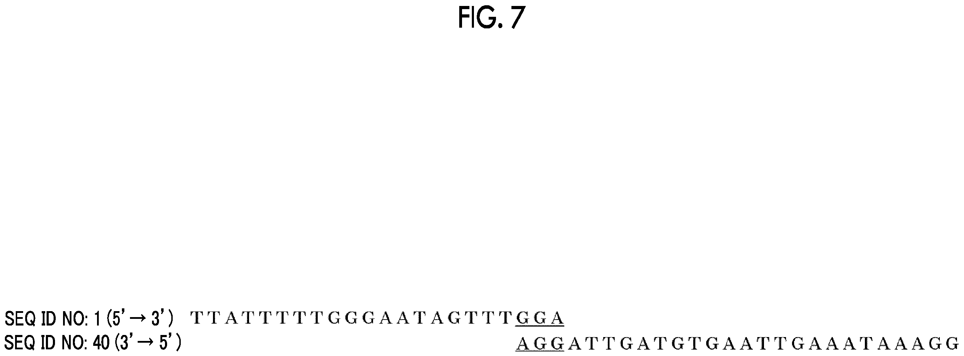

For example, as shown in Fig. 7, in the combination of primers of SEQ ID NO: 1 and SEQ ID NO: 40, the three bases from the 3' end (underlined portion) are an AG pair or a GG pair, which are not complementary to each other.

In a case where the above condition is satisfied, it is possible to prevent the primers from binding to each other at the 3' end side.

Note that Modification Example 3 can be combined with Modification Examples 1 and 2 described above. In addition, according to Modification Example 3, the aforementioned effect is additionally obtained.

### [Modification Example 4]

Next, a primer design device according to Modification Example 4 of the first embodiment of the present invention will be described. Regarding the primer design device according to Modification Example 4, the same processing as that of the first embodiment will not be described.

In the present embodiment, in the primer candidate sequence selection unit 30, the conditions (1) to (3) are set as predetermined selection conditions used for selecting primer candidate sequences. However, it is preferable that the predetermined selection conditions further include the following condition (7).

(7) The number of binding sites with the double-stranded genomic DNA before base conversion is equal to or less than a predetermined number.

The upper limit of the number of binding sites between the double-stranded genomic DNA before base conversion and the partial sequence is not particularly limited. From the viewpoint of markedly obtaining the desired effect of the present invention, the upper limit of the number of the aforementioned binding sites is preferably 5 or less, and particularly preferably 2 or less.

Note that Modification Example 4 can be combined with at least one of Modification Examples 1 to 3 described above.

### [Modification Example 5]

Next, a primer design device according to Modification Example 5 of the first embodiment of the present invention will be described. Regarding the primer design device according to Modification Example 5, the same processing as that of the first embodiment will not be described.

In Modification Example 1 of the first embodiment, in the primer candidate sequence selection unit 30, the conditions (1) to (3) are set as predetermined selection conditions used for selecting primer candidate sequences. However, it is preferable that the predetermined selection conditions further include the following condition (8).

(8) In a case where the predetermined number of YG sequences or CR sequences included in a partial sequence is set to 1 or more in the condition (2), a range of position of the YG sequences or CR sequences in the partial sequence is also specified, and the number of YG sequences or CR sequences included in the specified range of position is preferably equal to or less than a predetermined number.

"Range of position of YG sequences or CR sequences in a partial sequence" means where the YG sequences or CR sequences are located in the partial sequence. For example, it is possible to specify a predetermined range of position including the 5' end of the partial sequence and a predetermined range of position including the 3' end.

"Range of position" to be specified is not particularly limited. It is preferable to specify a range of position on the 5' end side of the partial sequence, because then the influence of methylation of cytosine (C) can be further reduced, compared to a case where a range of position on the 3' end side of the partial sequence is specified. More specifically, generally, base pair mismatches (non-complementary pairs) between a primer and a template DNA that occur on the 3' end side exert a higher influence compared to base pair mismatches that occur on the 5' end side, and hinder binding in many cases. In the present invention, in a case where a primer has a site of an uncertain base in the primer binding region, the site is preferably disposed on the 5' end side such that the presence of such a site does not hinder the binding of the primer.

Likewise, in Modification Example 2 of the first embodiment, in the primer candidate sequence selection unit 30, the conditions (1) to (4) are set as predetermined selection conditions used for selecting primer candidate sequences. However, it is preferable that the predetermined selection conditions further include the following condition (9).

(9) In a case where the predetermined number of YHG sequences or CDR sequences included in a partial sequence is set to 1 or more in the condition (4), a range of position of the YHG sequences or CDR sequences in the partial sequence is also specified, and the number of YHG sequences or CDR sequences included in the specified range of position is equal to or less than a predetermined number.

As in the condition (8), it is preferable to specify a range of position on the 5' end side of the partial sequence, because then the influence of methylation of cytosine (C) can be further reduced as described above, compared to a case where a range of position on the 3' end side of the partial sequence is specified.

Likewise, in Modification Example 3 of the first embodiment, in the primer candidate sequence selection unit 30, the conditions (1) to (3) and (5) are set as predetermined selection conditions used for selecting primer candidate sequences. However, it is preferable that the predetermined selection conditions further include the following condition (10).

(10) In a case where the predetermined number of YHH sequences or DDR sequences included in a partial sequence is set to 1 or more in the condition (5), a range of position of the YHH sequences or DDR sequences in the partial sequence is also specified, and the number of YHH sequences or DDR sequences included in the specified range of position is preferably equal to or less than a predetermined number.

As in the condition (8), it is preferable to specify a "range of position" on the 5' end side of the partial sequence, because then the influence of methylation of cytosine (C) can be further reduced as described above, compared to a case where a range of position on the 3' end side of the partial sequence is specified.

In the primer candidate sequence selection unit 30, as predetermined selection conditions used for selecting primer candidate sequences, the conditions (1) to (5) are set. The selection conditions may further include the conditions (8) to (10).

Note that Modification Example 5 can be combined with at least one of Modification Examples 3 and 4 described above. In addition, according to Modification Example 5, the aforementioned effect is additionally obtained.

### [Modification Example 6]

Next, a primer design device according to Modification Example 6 of the first embodiment of the present invention will be described. For the primer design device according to Modification Example 6, the same configuration as that in the first embodiment will be denoted by the same reference numeral, and the same processing as that in the first embodiment will not be described.

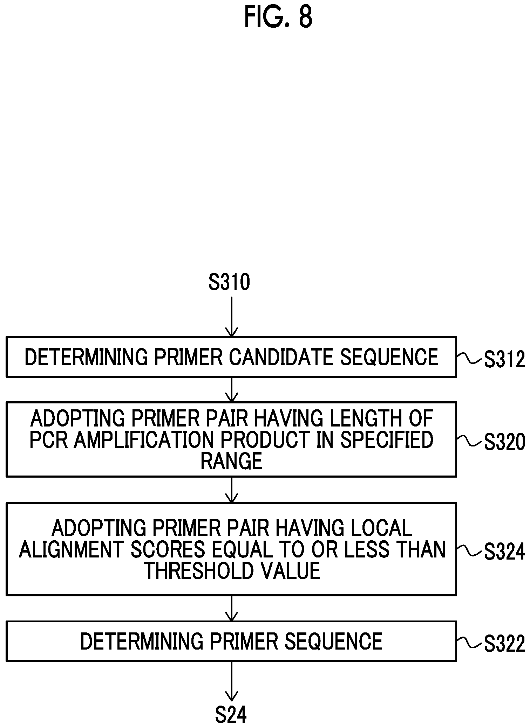

Fig. 8 is a flowchart showing a modification example of the condition B of Fig. 4, and the same processing as that in the first embodiment will not be described.

In the first embodiment, by the primer sequence determination unit 32, among one or more primer candidate sequences determined by the primer candidate sequence selection unit 30 (step S312), that is, among all the combinations of one or more forward primer candidate sequences of the first template strand (strand A+) and one or more reverse primer candidate sequences of the first template strand (strand A+) and all the combinations of one or more forward primer candidate sequences of the second template strand (strand B+) and one or more reverse primer candidate sequences of the second template strand (strand B+), a forward primer sequence and a reverse primer sequence to amplify the region including the target site selected by the partial sequence cutting unit 28 are adopted and determined based on the calculated length the PCR amplification products (step S320). However, the present embodiment is not limited thereto, and as shown in Fig. 8, the primer sequence determination unit 32 can adopt partial sequences as a forward primer sequence and a reverse primer sequence of the first template strand or the second template strand (step S320), then calculate local alignment scores for all combinations of the adopted primer sequences, determine whether or not the calculated local alignment scores are lower than a predetermined threshold value, adopt the primer sequences having local alignment scores calculated to be lower than the predetermined threshold value (step S324), and determine the sequences as primer sequences (step S322).

"All combinations of the adopted primer sequences" described above are not limited to combinations of a forward primer and a reverse primer, and include a combination of forward primer sequences, a combination of reverse primer sequences. Furthermore, "all combinations of the adopted primer sequences" are not limited to a forward primer sequence of the first template strand (strand A+) and a reverse primer sequence of the first template strand (strand A+), and include a combination of a forward primer sequence of the first template strand (strand A+) and a reverse primer sequence of the second template strand (strand B+), and the like.

The local alignment scores calculated under the aforementioned conditions are scores determined using scoring matrices set such that high scores are given in a case where bases are complementary to each other and low scores are given in a case where bases are not complementary to each other.

For example, a local alignment score calculation method for the combination of SEQ ID NO: 1 and SEQ ID NO: 40 shown in Fig. 8 will be described. In Fig. 8, "|" is attached to bases in a case where the bases of SEQ ID NO: 1 and SEQ ID NO: 40 are identical (complementary pair/match string), ":" is attached to bases in a case where the bases are not identical (non-complementary pair/mismatch string), and "-" is attached to gaps.

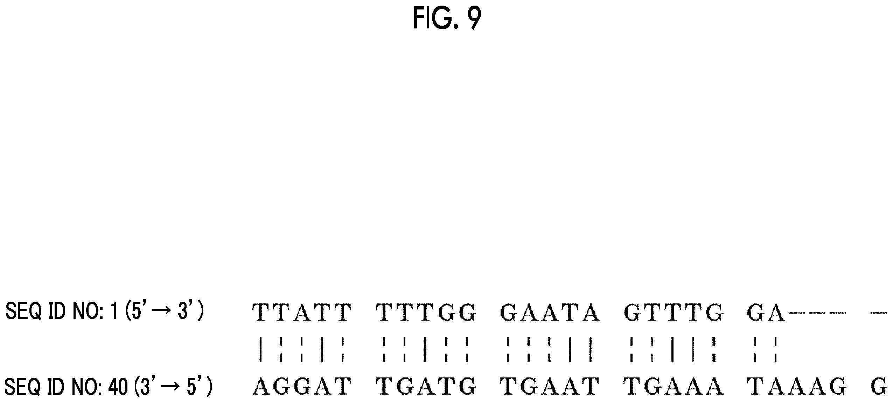

The sequences shown in Fig. 9 have 7 complementary pairs, 15 non-complementary pairs, and 4 gaps. Therefore, in a case where "+1" is given to the complementary pairs, "0" is given to the non-complementary pairs, and "-1" is given to the gaps, the pairwise alignment scores (local alignment scores) of the combination of SEQ ID NO: 1 and SEQ ID NO: 40 are calculated to be (+1 × 7) + (0 × 15) + (-1 × 4) = +3.

In a case where the above condition is satisfied, it is possible to avoid the influence of binding of primers to each other.

Note that Modification Example 6 can be combined with at least one of Modification Examples 1 to 5 described above. In addition, according to Modification Example 6, the aforementioned effect is additionally obtained.

### [Modification Example 7]

Next, a primer design device according to Modification Example 7 of the first embodiment of the present invention will be described. For the primer design device according to Modification Example 7, the same configuration as that in the first embodiment will be denoted by the same reference numeral, and the same processing as that in the first embodiment will not be described.

In the first embodiment, in order to amplify and analyze both strands of DNA, a device and a method for designing two sets of primers are described. However, the present invention is not limited thereto, and in a case where either of two DNA strands is to be analyzed, one set of primers may be designed. That is, although primers are designed based on the strand A and the strand B in Fig. 3B, primers may be designed based on only the strand A.

Furthermore, in a case where a DNA methylation maintenance mechanism is considered to be working, only one set of primers may be designed, because in a case where C in the CG sequence of one DNA strand is methylated, C in the CG sequence of the other strand is extremely highly likely to be methylated, and in a case where C in the CG sequence of one DNA strand is unmethylated, C in the CG sequence of the other strand is extremely highly likely to be unmethylated. When one set of primers cannot be designed based on one strand in this case, the primers may be designed based on the other strand.

In a case where only one set of primers is to be designed as described above, the complementary strand generation unit 26 produces only a complementary strand A- having a base sequence complementary to the base sequence of the strand A+ shown in Fig. 3C.

Then, the partial sequence cutting unit 28 selects one target site from one target site from one or more target sites acquired in the target site information acquisition unit 22 (step S280), detects "Y" of the selected target site or "R" (that is, a base which is in a methylation site in the target site) complementary to "Y" from the DNA sequences of the strand A+ and the strand A- based on the position information of the selected target site, cuts partial sequences as much as possible from partial sequences having a predetermined length from the base sequences ((1) and (2) in Fig. 3D) positioned on the 5' end side of the detected "Y" or "R" (step S282) to obtain one or more partial sequences.

The primer candidate sequence selection unit 30 is a unit that performs the primer candidate sequence selection step S20 shown in Fig. 2, and selects partial sequences satisfying all the predetermined selection conditions (1) to (3) as primer candidate sequences from one or more partial sequences of each strand cut out by the partial sequence cutting unit 28.

Among one or more partial sequences cut out from the first template strand (strand A+) (that is, one or more partial sequences cut out from (1) in Fig. 3D), a partial sequence that satisfies predetermined selection conditions is selected as a forward primer candidate sequence of the first template strand (strand A+). Among one or more partial sequences cut out from the first complementary strand (strand A-), a partial sequence that satisfies predetermined selection conditions is selected as a reverse primer candidate sequence of the first template strand (strand A+).

The primer sequence determination unit 32 acquires all primer pairs (combinations of a forward primer and a reverse primer) producible from the one or more selected forward primer candidate sequences of the first template strand (strand A+) and one or more reverse primer candidate sequences of the first template strand (strand A+), and calculates the lengths of PCR amplification products predicted to be amplified by PCR for each primer pair. Then, the primer sequence determination unit 32 determines whether or not the calculated length of each PCR amplification product is within a predetermined numerical range. In a case where the calculated length of the PCR amplification product is within a predetermined numerical range, the primer sequence determination unit 32 adopts the primer pair for which the length of the PCR amplification product is calculated (that is, a combination of a forward primer candidate sequence and a reverse primer candidate sequence) as a forward primer sequence and a reverse primer sequence of the first template strand to amplify the region including the target site selected by the partial sequence cutting unit 28 (partial sequence cutting step S18) and determines these sequences as a primer sequence.

Note that Modification Example 7 can be combined with at least one of Modification Examples 1 to 6 described above.

### [Second embodiment]

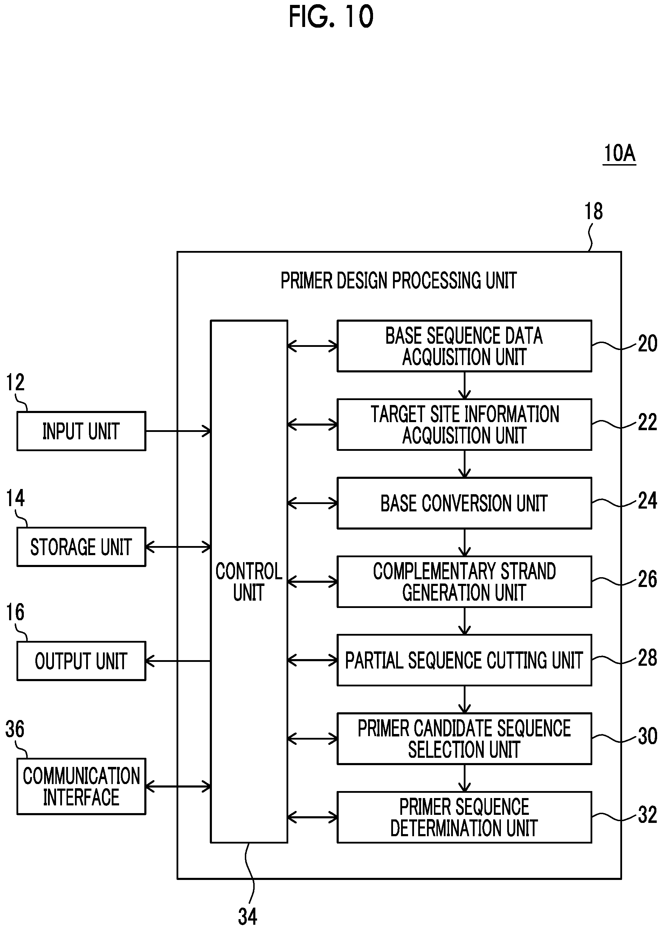

Fig. 10 is a block diagram conceptually showing an example a primer design device according to a second embodiment of the present invention. The device 10 of the first embodiment can also comprise a communication interface (communication device).

A primer design device 10A of the second embodiment shown in Fig. 10 has the same configuration as the primer design device 10 of the first embodiment shown in Fig. 1 except that the primer design device 10A has a communication interface 36. Therefore, the same configuration requirements are denoted by the same reference numerals and will not be described.

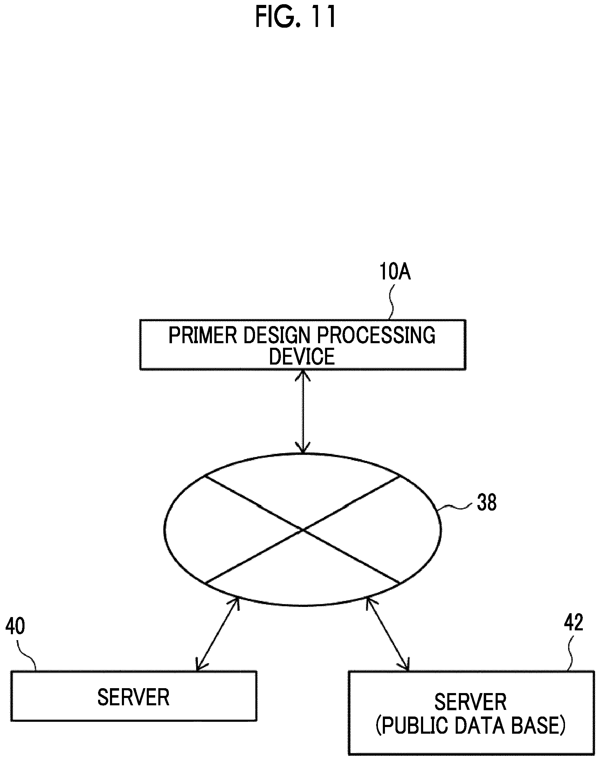

As shown in Fig. 11, via a communication network 38 such as the internet, the primer design device 10A can be connected to a server 42 comprising a public database installed on the outside of the device.

The device 10A of the present embodiment can operate at least one of the base sequence data acquisition unit 20, the target site information acquisition unit 22, the base conversion unit 24, the complementary strand generation unit 26, the partial sequence cutting unit 28, the primer candidate sequence selection unit 30, or the primer sequence determination unit 32 via the communication interface 36 according to the program located at the site of an external server 40. In this a case, a data processing device 10A of the present embodiment may not include the units operated according to the program in the external server.

For example, based on the instructions from the control unit 34, the communication interface 36 can acquire a DNA base sequence including genes and genomes from a public database via the communication network 38 and store the database in the storage unit 14. Examples of the public database include GenBank of the National Center for Biotechnology Information (NCBI) of the United States, ENA of the European Molecular Biology Laboratory (EMBL), and DDBJ of National Institute of Genetics, and the like.

The base sequence acquired from the public database may be a partial sequence of the base sequence of the genomic DNA of biological species for which a primer is to be designed. The base sequence is preferably a complete sequence.

For example, based on the instructions from the control unit 34, the communication interface 36 can search for the identity of sequences via the communication interface 36 by using a public search server 40 to perform binding determination relating to the condition 8 in the primer candidate sequence selection unit 30, local alignment search of the primer sequence determination unit 32 in Modification Example 6, and the like, via the communication network 38. Examples of the public search server include BLAST of the National Center for Biotechnology Information (NCBI) of the United States and the like.

### [Third embodiment]

A third embodiment is a method of manufacturing a primer by synthesizing a primer based on the primer sequence designed by the primer design device and the primer design method according to the first and second embodiments.

The primer design method is as shown in the first and second embodiments.

Known methods can be used as the primer synthesis method. Examples thereof include a method of chemically synthesizing a primer from terminal bases with a DNA synthesizer or an RNA synthesizer by using deoxyribonucleoside triphosphate (dNTP) or the like as a material. Commercially available products can be used as the synthesizer.

In the device according to an embodiment of the present invention, each configuration requirements included in the device may be configured with the dedicated hardware or may be configured with a programmed computer.

The method according to an embodiment of the present invention can be performed by, for example, a program for causing a computer to execute each step of the method. It is also possible to provide a computer-readable recording medium on which this program is recorded.

Hitherto, the present invention has been specifically described. However, the present invention is not limited to the above embodiments. It goes without saying that various types of amelioration or modification may be added thereto without departing from the gist of the present invention.

### Examples

### [Example 1 and Comparative Example 1]

Based on the base sequence data of reference genome GRCh37 (GenBank assembly accession: GCA_000001405.1, RefSeq assembly accession: GCF_000001405.13), 50 measurement sites (target sites) shown in Table 1, and the position information on the target sites, a primer for multiplex PCR producing a PCR amplification product having a length of 100 bp to 300 bp was designed using the primer design device of the first embodiment.

The primer was designed such that the primer had a length of 20 to 30 bases (mer), and that only C in a CG sequence can be methylated.

In addition, the conditions of Example 1 for determining the partial sequence were set as follows.

Condition (1): The Tm value is in a range of 55°C to 60°C.

Condition (2): The number of YG sequences or CR sequences included in a partial sequence is 0.

Condition (3): The upper limit of the number of binding sites with the sequence outside the related region is 2.

Meanwhile, the conditions of Comparative Example 1 for determining the partial sequence were set as follows.

Condition (1): The Tm value is in a range of 55°C to 60°C.

Condition (2): The number of YG sequences or CR sequences included in a partial sequence is 0.

Condition (3): The number of binding sites with the sequence outside the related region is 0.

Table 1 shows whether the primer for each measurement site of Example 1 and Comparative Example 1 is successfully designed or failed to be designed and shows the primer design success rate calculated from the results of the success or failure of the primer design. In addition, Table 2 shows the primers that could be designed in Example 1, and Table 3 shows the primers that could be designed in Comparative Example 1.

As shown in Table 1, in the example, primers related to the target sites of ID9, 19, 28, and 50 could be designed, but in Comparative Example 1, primers related to these target sites could not be designed.

The design success rate was 78% in Example 1 and 70% in Comparative Example 1, which shows that performing determination on partial sequences under the condition (3) increases the design success rate.

**[Table 1]**

| Measurement site | | | Success or failure of design | |
|---|---|---|---|---|
| ID | Chromosome | Coordinate | Example 1 | Comparative Example 1 |
| 1 | 16 | 53468112 | X | X |
| 2 | 3 | 37459206 | X | X |
| 3 | 3 | 171916037 | X | X |
| 4 | 1 | 91194674 | X | X |
| 5 | 8 | 42263294 | X | X |
| 6 | 14 | 69341139 | X | X |
| 7 | 16 | 28890100 | X | X |
| 8 | 8 | 41167802 | X | X |
| 9 | 1 | 230560793 | X | - |
| 10 | 6 | 25282779 | X | X |
| 11 | 2 | 23913414 | X | X |
| 12 | 4 | 154609857 | X | X |
| 13 | 11 | 2720463 | X | X |
| 14 | 8 | 49890609 | - | - |
| 15 | 1 | 5937253 | X | X |
| 16 | 8 | 87081553 | X | X |
| 17 | 3 | 15106710 | - | - |
| 18 | 14 | 60389786 | X | X |
| 19 | 20 | 48959004 | X | - |
| 20 | 1 | 214170376 | X | X |
| 21 | 1 | 43831041 | X | X |
| 22 | 19 | 54746945 | - | - |
| 23 | 1 | 51034865 | X | X |
| 24 | 10 | 80828702 | X | X |
| 25 | 1 | 200011786 | - | - |
| 26 | 1 | 170490434 | X | X |
| 27 | 2 | 103414042 | X | X |
| 28 | 12 | 11905390 | X | - |
| 29 | 16 | 47008491 | X | X |
| 30 | 10 | 69990588 | X | X |
| 31 | 18 | 60055084 | X | X |
| 32 | 1 | 20960010 | - | - |
| 33 | 21 | 30390417 | X | X |
| 34 | 17 | 79670410 | - | - |
| 35 | 2 | 238249536 | X | X |
| 36 | 8 | 19555033 | X | X |
| 37 | 8 | 144120399 | - | - |
| 38 | 3 | 57743543 | X | X |
| 39 | 10 | 28961565 | X | X |
| 40 | 8 | 120428418 | - | - |
| 41 | 2 | 218988876 | X | X |
| 42 | 4 | 2264510 | - | - |
| 43 | 7 | 127721794 | X | X |
| 44 | 3 | 52870543 | X | X |
| 45 | 1 | 1268793 | - | - |
| 46 | 16 | 31437783 | X | X |
| 47 | 1 | 153515502 | X | X |
| 48 | 12 | 56583610 | - | - |
| 49 | 1 | 169396706 | X | X |
| 50 | 17 | 57839538 | X | - |
| | | Design success rate | 78% | 70% |

| | | | | |
|---|---|---|---|---|
| X: success of design, -: failure of design | | | | |

**[Table 2]**

| Primer that could be designed in Example 1 | | | | | | |
|---|---|---|---|---|---|---|
| Measurement site | Forward primer | | | Reverse primer | | |
| ID | Name | Base sequence (5'-3') | SEQ ID NO: | Name | Base sequence (5'-3') | SEQ ID NO: |
| 1 | 1F1 | TTATTTTTGGGAATAGTTTGGA | 1 | 1R1 | TAACTCCCCTAATTTCACCA | 40 |
| 2 | 2F1 | TTTTAATTATTATTTTATTTTGAAGA | 2 | 2R1 | CCTACTCACCTATTACCCCA | 41 |
| 3 | 3F1 | GAGTATATTTTTTTTGGTTTAAAGA | 3 | 3R1 | AAATATTCTCCAACTCTATCCAC | 42 |
| 4 | 4F1 | GGAAATAAAGTTAAGTGTAGTTAGGA | 4 | 4R1 | TTCTATTTCATTTCAAAAAAACA | 43 |
| 5 | 5F1 | TTTTTAAAATAAATTATATTAAGGTAAGAA | 5 | 5R1 | TCTAACCCACCAATTTATACA | 44 |
| 6 | 6F1 | GATAAATGGAAAAAAAGAGGAA | 6 | 6R1 | AACTAACAAACACACATACACCA | 45 |
| 7 | 7F1 | TAGAGTTTTGTGGAGGGAAGA | 7 | 7R1 | CTCTTCCAAAAAACACTTACCA | 46 |
| 8 | 8F1 | TTTTAATTAATAGGGGTTTAGGA | 8 | 8R1 | TACCCCAATATTAAAAACCAC | 47 |
| 9 | 9F1 | TGTGGTTATAGTGGTAAGTAGGA | 9 | 9R1 | TTCACTAACAACCCAAAAACA | 48 |
| 10 | 10F1 | GATAAGATAGGAGTTTTGTGAAGA | 10 | 10R1 | CACACTAAATAAAACATATAATTTAACA | 49 |
| 11 | 11F1 | TAGTTTTATTTTTTTATTTTGTAGAAGA | 11 | 11R1 | AAATAACCTCTATAATCCACTCAC | 50 |
| 12 | 12F1 | TTGGTTAGATGTTTGTAGTAGGA | 12 | 12R1 | AACTAACCCCAAATTTAACCA | 51 |
| 13 | 13F1 | ATTTAGTTTATTTTGAATTATTATGAGA | 13 | 13R1 | TCCCATCTACACCTTATAAACA | 52 |
| 15 | 15F1 | AATTATGTGGGAGGTAGGGA | 14 | 15R1 | AACTAACTACCATACTACTAACCCA | 53 |
| 16 | 16F1 | TAGTGGAAAAGAATGGGAGA | 15 | 16R1 | TTCAACTTAACTCAATTTTAAACA | 54 |
| 18 | 18F1 | TAAATGGGTTAGTTTTGAAAGA | 16 | 18R1 | ACAATTTAATTTAAAAACCCCA | 55 |
| 19 | 19F1 | TAGGTAGGGTGGAAAAAAGAA | 17 | 19R1 | AATACAAAATAATCTAACCCCA | 56 |
| 20 | 20F1 | GAGGATATATGTAGTAATTTTTTAAGAGA | 18 | 20R1 | TTCATTACCCCTTAATACCA | 57 |
| 21 | 21F1 | TTAGTAAGGGGGATTTTATTAGA | 19 | 21R1 | AAACTTATACCAAAAAATAATAAAACA | 58 |
| 23 | 23F1 | TGGTAATAAAATGGTGAATAAGA | 20 | 23R1 | ATACTAATAAAAAAAAATAAAAACCCA | 59 |
| 24 | 24F1 | AATGTTTATAGATAGTGAAAAGGAA | 21 | 24R1 | AAAATAAAATAACCAATATAATCTAACAC | 60 |
| 26 | 26F1 | AGTGAAGATAGTATAAGTAAAAGATAGGA | 22 | 26R1 | AAAAAACTAAACTAAACTAAACAAACA | 61 |
| 27 | 27F1 | AATTGGGTATTTAAATGGGAGA | 23 | 27R1 | ACCAAAAATAAATCTAAACAAACA | 62 |
| 28 | 28F1 | TTTTTGTTTTTATTTTTAATAGTAGGA | 24 | 28R1 | CCTCTCTAACCTAAAACCCAC | 63 |
| 29 | 29F1 | TTTTTATAATTAGGTTAAAGGGAA | 25 | 29R1 | ATATAAAACAAATCTAACCACCA | 64 |
| 30 | 30F1 | TTTTGAGGGAAAATAATTTTAAGAA | 26 | 30R1 | CTTCATACTTACACACACAATACA | 65 |
| 31 | 31F1 | TGATTTAATTAAAGTTTATAGTAATTTTGA | 27 | 31R1 | ATCACCATTAACCAAACACA | 66 |
| 33 | 33F1 | ATTTAAAGGTTTTGAGGAAGGA | 28 | 33R1 | ATAATCCTATCAAAACCACCA | 67 |
| 35 | 35F1 | TTTGGAGTTATAGTTAGTTAGGGA | 29 | 35R1 | CAAACAACTAAACATAAACCCA | 68 |
| 36 | 36F1 | TGATGGAAATTTAGGTAGAAGA | 30 | 36R1 | ATCTCTAAATTTTTCCAACCA | 69 |
| 38 | 38F1 | TTGATTGTAAAGTGTTATTAAGGA | 31 | 38R1 | CTCTATTTTCCATTCAACCA | 70 |
| 39 | 39F1 | TTAGTTAGAATTTTATTTAAGTTTAAGAGA | 32 | 39R1 | CCAAACATAAATCCCCTATACA | 71 |
| 41 | 41F1 | TTTTGGTTTTTAGGAGTGAAGA | 33 | 41R1 | AAACAATAAACCTTCCCACA | 72 |
| 43 | 43F1 | GGAATAATGTTTAGGTAGGTAGTTTAGGA | 34 | 43R1 | CATCCTACCCAACATAACCA | 73 |
| 44 | 44F1 | AGGGATTAGAGTGAGAAGGAA | 35 | 44R1 | TATACTCTACCTCCCCAACA | 74 |
| 46 | 46F1 | TGGAAGAGTTATAATTTAGGGA | 36 | 46R1 | ACCCTCTCCCCTAATACACA | 75 |
| 47 | 47F1 | TTAGGGATTTTTATGGTAGAGA | 37 | 47R1 | CAAATCATTAACTTCAAACCAC | 76 |
| 49 | 49F1 | AGTTTTTTATATTGAGGTAAAGGA | 38 | 49R1 | AAAAATTTCACAACCAACAC | 77 |
| 50 | 50F1 | GGTATGGAATGTGTTTATAGATAAGA | 39 | 50R1 | CAAATTCCAACTCTTATAAAAACA | 78 |

**[Table 3]**

| Primer that could be designed in Comparative Example 1 | | | | | | |
|---|---|---|---|---|---|---|
| Measure ment site | Forward primer | | | Reverse primer | | |
| ID | Name | Base sequence (5'→3') | SEQ ID NO: | Name | Base sequence (5'→3') | SEQ ID NO: |
| 1 | 1F2 | TTATTTTTGGGAATAGTTTGGA | 79 | 1R2 | TAACTCCCCTAATTTCACCA | 114 |
| 2 | 2F2 | TTTTAATTATTATTTTATTTTGAAGGA | 80 | 2R2 | CCTACTCACCTATTACCCCA | 115 |
| 3 | 3F2 | GAGTATATTTTTTTTTGGTTTAAAGA | 81 | 3R2 | AAATATTCTCCAACTCTATCCAC | 116 |
| 4 | 4F2 | GGAAATAAAGTTAAGTGTAGTTAGG A | 82 | 4R2 | TTTCTATTTCATTTCAAAAAAACA | 117 |
| 5 | SF2 | TTTTTAAAATAAATTATATTAAGGTA AGAA | 83 | 5R2 | TCTAACCCACCAATTTATACA | 118 |
| 6 | 6F2 | GATAAATGGAAAAAAAGAGGAA | 84 | 6R2 | AACTAACAAACACACATACACCA | 119 |
| 7 | 7F2 | GGGGGTAGGAGAGTAAAAAGA | 85 | 7R2 | TAAAAATACAAAAAAACATTAATTACCA | 120 |
| 8 | SF2 | TTTTAATTAATAGGGGTTTAGGA | 86 | 8R2 | ACTACCCCAATATTAAAAACCA | 121 |
| 10 | 10F2 | GATAAGATAGGAGTTTTGTGAAGA | 87 | 10R2 | CACACTAAATAAAACATATAATTTAACA | 122 |
| 11 | 11F2 | TAGTTTTATTTTTTTATTTTGTAGAAG A TTGGTTTAGATGTTTGTAGTAGGA | 88 | 11R2 | ACTAAAAAATAACCTCTATAATCCAC | 123 |
| 12 | 12F2 | TTGGTTAGATGTTTGTAGTAGGA | 89 | 12R2 | AACACTATAACATATTTATAACAACCA | 124 |
| 13 | 13F2 | ATTTAGTTTATTTTGAATTATTATGAG A | 90 | 13R2 | TCCCATCTACACCTTATAAACA | 125 |
| 15 | 15F2 | AATTATGTGGGAGGTAGGGA | 91 | 15R2 | AACTAACTACCATACTACTAACCCA | 126 |
| 16 | 16F2 | TAGTGGAAAAGAATGGGAGA | 92 | 16R2 | TTCAACTTAACTCAATTTTAAACA | 127 |
| 18 | 18F2 | TAAATGGGTTAGTTTTGAAAGA | 93 | 18R2 | TACAATTTAATTTAAAAACCCCA | 128 |
| 20 | 20F2 | GAGGATATATGTAGTAATTTTTTAAG AGA | 94 | 20R2 | CATTACCCCTTAATACCACA | 129 |
| 21 | 21F2 | TTAGTAAGGGGGATTTTATTAGA | 95 | 21R2 | AAACTTATACCAAAAAATAATAAAACA | 130 |
| 23 | 23F2 | TGGTAATAAAATGGTGAATAAGA | 96 | 23R2 | AATACTAATAAAAAAAAATAAAAACCCA | 131 |
| 24 | 24F2 | AATGTTTATAGATAGTGAAAAGGAA | 97 | 24R2 | AAAATAAAATAACCAATATAATCTAACAC | 132 |
| 26 | 26F2 | AGTGAAGATAGTATAAGTAAAAGAT AGGA | 98 | 26R2 | ATAAATAAATAAATCAAACTAATAACCA | 133 |
| 27 | 27F2 | AATTGGGTATTTAAATGGGAGA | 99 | 27R2 | TACCAAAAATAAATCTAAACAAACA | 134 |
| 29 | 29F2 | TTTTTATAATTAGGTTAAAGGGAA | 100 | 29R2 | ACCACCAAATAACTTAATTTACA | 135 |
| 30 | 30F2 | TTTTGAGGGAAAATAATTTTAAGAA | 101 | 30R2 | CTTCATACTTACACACACAATACA | 136 |
| 31 | 31F2 | TGATTTAATTAAAGTTTATAGTAATTT TGA | 102 | 31R2 | AATAATCACCATTAACCAAACA | 137 |
| 33 | 33F2 | TTATGGAGAAAAGTGAAAGGA | 103 | 33R2 | TAAAACTCACCAACTTAACCA | 138 |
| 35 | 35F2 | TTTGGAGTTATAGTTAGTTAGGGA | 104 | 35R2 | ATCAAACAACTAAACATAAACCCA | 139 |
| 36 | 36F2 | AAAGTTTGGTTATTATTTTTGTAAGA | 105 | 36R2 | CTTAAACTCCTTCAAATCCAC | 140 |
| 38 | 38F2 | TTGATTGTAAAGTGTTATTAAGGA | 106 | 38R2 | CTCTATTTTCCATTCAACCA | 141 |
| 39 | 39F2 | TTAGTTAGAATTTTATTTAAGTTTAA GAGA | 107 | 39R2 | CCAAACATAAATCCCCTATACA | 142 |
| 41 | 41F2 | TTTTGGTTTTTAGGAGTGAAGA | 108 | 41R2 | AATTAAATAAATAAATAAAAAATACCCCA | 143 |
| 43 | 43F2 | GGAATAATGTTTAGGTAGTTTAGGA | 109 | 43R2 | CATCCTACCCAACATAACCA | 144 |
| 44 | 44F2 | AGGGATTAGAGTGAGAAGGAA | 110 | 44R2 | TATACTCTACCTCCCCAACA | 145 |
| 46 | 46F2 | TGGAAGAGTTATAATTTAGGGA | 111 | 46R2 | CTCCTAAAACAACCTACCCTAC | 146 |
| 47 | 47F2 | TTAGGGATTTTTATGGTAGAGA | 112 | 47R2 | CAAATCATTAACTTCAAACCAC | 147 |
| 49 | 49F2 | AGTTTTTTATATTGAGGTAAAGGA | 113 | 49R2 | AAATTTCACAACCAACACA | 148 |

| | | | | | | |
|---|---|---|---|---|---|---|
| Explanation of References | | | | | | |

10, 10A: Primer design device
12: Input unit
14: Storage unit
16: Output unit
18: Primer design processing unit
20: Base sequence data acquisition unit
22: Target site information acquisition unit
24: Base conversion unit
26: Complementary strand generation unit
28: Partial sequence cutting unit
30: Primer candidate sequence selection unit
32: Primer sequence determination unit
34: Control unit
36: Communication interface
38: Communication network
40, 42: Server

The primer designed according to the present invention can be used for measuring the DNA methylation degree of a biological sample in the fields of drug discovery, diagnosis, and other bioindustries.
[Sequence list] International application 20F00959W1JP21042153_3.app based on the Patent Cooperation Treaty

## Claims

1. A primer design method for amplicon methylation sequence analysis that is a method for designing a primer used to simultaneously amplify a plurality of regions each including one or more target sites for measuring a methylation degree by using a bisulfite reaction or an enzyme reaction and multiplex PCR to measure a methylation degree of double-stranded genomic DNA in a predetermined site related to a predetermined biological phenomenon, the method comprising:
a base sequence data acquisition step of acquiring base sequence data of the double-stranded genomic DNA;
a target site information acquisition step of acquiring the one or more target sites and position information thereof;
a base conversion step of converting methylatable "C" into "Y" and converting other "C" into "T" in the base sequence data of the double-stranded genomic DNA;
a complementary strand generation step of generating a complementary strand for each template strand of the double-stranded genomic DNA after base conversion;
a partial sequence cutting step of selecting one target site from the one or more target sites and cutting one or more partial sequences from each strand based on the position information of the selected target site, the one or more partial sequences having a predetermined length from a base sequence positioned on the 5' end side of "Y" formed as a result of conversion of the selected target site or "R" complementary to "Y";
a primer candidate sequence selection step of selecting partial sequences that satisfy predetermined selection conditions as primer candidate sequences from the one or more partial sequences cut out from each strand;
a primer sequence determination step of adopting and determining a forward primer sequence and a reverse primer sequence to amplify a region including the selected target site cut out from each template strand, from the one or more selected primer candidate sequences; and
a repetition step of repeating the partial sequence cutting step, the primer candidate sequence selection step, and the primer sequence determination step until all of the one or more target sites are selected in the partial sequence cutting step,
wherein the methylatable "C" is "C" in a CG sequence, and
the predetermined selection conditions include (1) a Tm value is within a predetermined range, (2) the number of YG sequences or CR sequences included in a partial sequence is equal to or less than a predetermined number, and (3) an upper limit of the number of binding sites with a sequence outside a related region on the double-stranded genomic DNA after base conversion is equal to or less than a predetermined number that is equal to or more than 1 [where "C", "G", "Y", and "R" are base codes established by IUPAC, "C" represents cytosine, "G" represents guanine, "Y" represents thymine or cytosine, and "R" represents adenine or guanine].

2. The primer design method according to claim 1,
wherein the methylatable "C" further includes "C" in a CHG sequence, and
the predetermined selection conditions further include (4) the number of YHG sequences or CDR sequences included in the partial sequence is equal to or less than a predetermined number [where "C", "G", "Y", "H", "R", and "D" are base codes established by IUPAC, "C" represents cytosine, "G" represents guanine, "Y" represents thymine or cytosine, "H" represents adenine, cytosine, or thymine, "D" represents thymine, guanine, or adenine, and "R" represents adenine or guanine].

3. The primer design method according to claim 1 or 2,
wherein the methylatable "C" further includes "C" in a CHH sequence, and
the predetermined selection conditions further include (5) the number of YHH sequences or DDR sequences included in the partial sequence is equal to or less than a predetermined number [where "Y", "H", "R", and "D" are base codes established by IUPAC, "Y" represents thymine or cytosine, "H" represents adenine, cytosine, or thymine, "D" represents thymine, guanine, or adenine, and "R" represents adenine or guanine].

4. The primer design method according to any one of claims 1 to 3,
wherein the predetermined selection conditions further include (6) three bases from the 3' end of the partial sequence are not complementary to three bases from the 3' end of the other partial sequence.

5. The primer design method according to any one of claims 1 to 4,
wherein the predetermined selection conditions further include (7) the number of binding sites with the double-stranded genomic DNA before base conversion is equal to or less than a predetermined number.

6. The primer design method according to any one of claims 1 to 5,
wherein the predetermined selection conditions further include (8) in a case where the predetermined number of YG sequences or CR sequences included in the partial sequence is set to 1 or more in the condition (2), a range of position of the YG sequences or CR sequences in the partial sequence is also specified, and the number of the YG sequences or CR sequences included in the specified range of position is equal to or less than a predetermined number.

7. The primer design method according to any one of claims 2 to 6,
wherein the predetermined selection conditions further include (9) in a case where the predetermined number of YHG sequences or CDR sequences included in the partial sequence is set to 1 or more in the condition (4), a range of position of the YHG sequences or CDR sequences in the partial sequence is also specified, and the number of the YHG sequences or CDR sequences included in the specified range of position is equal to or less than a predetermined number.

8. The primer design method according to any one of claims 3 to 7,
wherein the predetermined selection conditions further include (10) in a case where the predetermined number of YHH sequences or DDR sequences included in the partial sequence is set to 1 or more in the condition (5), a range of position of the YHH sequences or DDR sequences in the partial sequence is also specified, and the number of the YHH sequences or DDR sequences included in the specified range of position is equal to or less than a predetermined number.

9. The primer design method according to any one of claims 1 to 8,
wherein the primer candidate sequence selection step is a step of dividing the double-stranded genomic DNA after the base conversion into a first template strand and a second template strand, adopting a complementary strand of the first template strand as a first complementary strand, adopting a complementary strand of the second template strand as a second complementary strand, selecting a partial sequence satisfying predetermined selection conditions as a forward primer candidate sequence of the first template strand among one or more partial sequences cut out from the first template strand, selecting a partial sequence satisfying the predetermined selection conditions as a reverse primer candidate sequence of the first template strand among one or more partial sequences cut out from the first complementary strand, selecting a partial sequence satisfying the predetermined selection conditions as a forward primer candidate sequence of the second template strand among one or more partial sequences cut out from the second template strand, and selecting a partial sequence satisfying the predetermined selection conditions as a reverse primer candidate sequence of the second template strand among one or more partial sequences cut out from the second complementary strand.

10. The primer design method according to claim 9,
wherein the primer sequence determination step is a step of calculating a length of a PCR amplification product predicted to be amplified by PCR for all combinations of the one or more forward primer candidate sequences of the first template strand and the one or more reverse primer candidate sequences of the first template strand selected in the primer candidate sequence selection step, adopting a combination of primer candidate sequences for which the length of the PCR amplification product is calculated to be within a predetermined range as a forward primer sequence and a reverse primer sequence of the first template strand to amplify a region including the target site selected in the partial sequence cutting step, calculating a length of a PCR amplification product predicted to be amplified by PCR for all combinations of the one or more forward primer candidate sequences of the second template strand and the one or more reverse primer candidate sequences of the second template strand selected in the primer candidate sequence selection step, and adopting a combination of primer candidate sequences for which the length of the PCR amplification product is calculated to be within a predetermined range as a forward primer sequence and a reverse primer sequence of the second template strand to amplify a region including the target site selected in the partial sequence cutting step.

11. The primer design method according to any one of claims 1 to 10,
wherein after the forward primer sequence and the reverse primer sequence are adopted for all target sites, the primer sequence determination step further calculates local alignment scores for all combinations of the adopted primer sequences and adopts and determines a combination for which the local alignment scores are calculated to be lower than a predetermined threshold value as a primer sequence.

12. The primer design method according to any one of claims 1 to 11,
wherein in the condition (3), the upper limit of the number of binding sites with the partial sequence is 1 or 2.

13. A primer manufacturing method comprising:
a primer design step; and
a synthesis step of synthesizing a primer based on a primer sequence designed in the primer design step,
wherein the primer design step is performed by the primer design method according to any one of claims 1 to 12.

14. A primer design device for amplicon methylation sequence analysis that is a device for designing a primer used to simultaneously amplify a plurality of regions each including one or more target sites for measuring a methylation degree by using a bisulfite reaction or an enzyme reaction and multiplex PCR to measure a methylation degree of double-stranded genomic DNA in a predetermined site related to a predetermined biological phenomenon, the design device comprising:
a base sequence data acquisition unit that acquires base sequence data of the double-stranded genomic DNA;
a target site information acquisition unit that acquires the one or more target sites and position information thereof;
a base conversion unit that converts methylatable "C" into "Y" and converting other "C" into "T" in the base sequence data of the double-stranded genomic DNA;
a complementary strand generation unit that generates a complementary strand for each template strand of the double-stranded genomic DNA after base conversion;
a partial sequence cutting unit that selects one target site from the one or more target sites and cuts one or more partial sequences from each strand based on the position information of the selected target site, the one or more partial sequences having a predetermined length from a base sequence positioned on the 5' end side of "Y" formed as a result of conversion of the selected target site or "R" complementary to "Y";
a primer candidate sequence selection unit that selects partial sequences satisfying predetermined selection conditions as primer candidate sequences from the one or more partial sequences cut out from each strand;
a primer sequence determination unit that adopts and determines a forward primer sequence and a reverse primer sequence to amplify a region including the selected target site cut out from each template strand, from the one or more selected primer candidate sequences; and
a control unit that controls the partial sequence cutting unit, the primer candidate sequence selection unit, and the primer sequence determination unit such that each of these units repeat processing thereof until all of the one or more target sites are selected in the partial sequence cutting unit,
wherein the methylatable "C" is "C" in a CG sequence, and
the predetermined selection conditions include (1) Tm is within a predetermined range, (2) the number of YG sequences or CR sequences included in a partial sequence is equal to or less than a predetermined number, and (3) an upper limit of the number of binding sites with a sequence outside a related region on the double-stranded genomic DNA after base conversion is equal to or less than a predetermined number that is equal to or more than 1 [where "C", "G", "Y", and "R" are base codes established by IUPAC, "C" represents cytosine, "G" represents guanine, "Y" represents thymine or cytosine, and "R" represents adenine or guanine].

15. The primer design device according to claim 14,
wherein the methylatable "C" further includes "C" in a CHG sequence, and
the predetermined selection conditions further include (4) the number of YHG sequences or CDR sequences included in the partial sequence is equal to or less than a predetermined number [where "C", "G", "Y", "H", "R", and "D" are base codes established by IUPAC, "C" represents cytosine, "G" represents guanine, "Y" represents thymine or cytosine, "H" represents adenine, cytosine, or thymine, "D" represents thymine, guanine, or adenine, and "R" represents adenine or guanine].

16. The primer design device according to claim 14 or 15,
wherein the methylatable "C" further includes "C" in a CHH sequence, and
the predetermined selection conditions further include (5) the number of YHH sequences or DDR sequences included in the partial sequence is equal to or less than a predetermined number [where "Y", "H", "R", and "D" are base codes established by IUPAC, "Y" represents thymine or cytosine, "H" represents adenine, cytosine, or thymine, "D" represents thymine, guanine, or adenine, and "R" represents adenine or guanine].

17. The primer design device according to any one of claims 14 to 16,
wherein the predetermined selection conditions further include (6) three bases from the 3' end of the partial sequence are not complementary to three bases from the 3' end of the other partial sequence.

18. The primer design device according to any one of claims 14 to 17,
wherein the predetermined selection conditions further include (7) the number of binding sites with the double-stranded genomic DNA before base conversion is equal to or less than a predetermined number.

19. The primer design device according to any one of claims 14 to 18,
wherein the predetermined selection conditions further include (8) in a case where the predetermined number of YG sequences or CR sequences included in the partial sequence is set to 1 or more in the condition (2), a range of position of the YG sequences or CR sequences in the partial sequence is also specified, and the number of the YG sequences or CR sequences included in the specified range of position is equal to or less than a predetermined number.

20. The primer design device according to any one of claims 15 to 19,
wherein the predetermined selection conditions further include (9) in a case where the predetermined number of YHG sequences or CDR sequences included in the partial sequence is set to 1 or more in the condition (4), a range of position of the YHG sequences or CDR sequences in the partial sequence is also specified, and the number of the YHG sequences or CDR sequences included in the specified range of position is equal to or less than a predetermined number.

21. The primer design device according to any one of claims 16 to 20,
wherein the predetermined selection conditions further include (10) in a case where the predetermined number of YHH sequences or DDR sequences included in the partial sequence is set to 1 or more in the condition (5), a range of position of the YHH sequences or DDR sequences in the partial sequence is also specified, and the number of the YHH sequences or DDR sequences included in the specified range of position is equal to or less than a predetermined number.

22. The primer design device according to any one of claims 14 to 21,
wherein the primer candidate sequence selection unit divides the double-stranded genomic DNA after the base conversion into a first template strand and a second template strand, adopts a complementary strand of the first template strand as a first complementary strand, adopts a complementary strand of the second template strand as a second complementary strand, selects a partial sequence satisfying predetermined selection conditions as a forward primer candidate sequence of the first template strand among one or more partial sequences cut out from the first template strand, selects a partial sequence satisfying the predetermined selection conditions as a reverse primer candidate sequence of the first template strand among one or more partial sequences cut out from the first complementary strand, selects a partial sequence satisfying the predetermined selection conditions as a forward primer candidate sequence of the second template strand among one or more partial sequences cut out from the second template strand, and selects a partial sequence satisfying the predetermined selection conditions as a reverse primer candidate sequence of the second template strand among one or more partial sequences cut out from the second complementary strand.

23. The primer design device according to claim 22,
wherein primer sequence determination unit calculates a length of a PCR amplification product predicted to be amplified by PCR for all combinations of the one or more forward primer candidate sequences of the first template strand and the one or more reverse primer candidate sequences of the first template strand selected in the primer candidate sequence selection unit, adopts a combination of primer candidate sequences for which the length of the PCR amplification product is calculated to be within a predetermined range as a forward primer sequence and a reverse primer sequence of the first template strand to amplify a region including the target site selected in the partial sequence cutting unit, calculates a length of a PCR amplification product predicted to be amplified by PCR for all combinations of the one or more forward primer candidate sequences of the second template strand and the one or more reverse primer candidate sequences of the second template strand selected in the primer candidate sequence selection unit, and adopts a combination of primer candidate sequences for which the length of the PCR amplification product is calculated to be within a predetermined range as a forward primer sequence and a reverse primer sequence of the second template strand to amplify a region including the target site selected in the partial sequence cutting unit.

24. The primer design device according to any one of claims 14 to 23,
wherein after the forward primer sequence and the reverse primer sequence are adopted for all target sites, the primer sequence determination unit further calculates local alignment scores for all combinations of the adopted primer sequences and adopts and determines a combination for which the local alignment scores are calculated to be lower than a predetermined threshold value as a primer sequence.

25. The primer design device according to any one of claims 14 to 24,
wherein in the condition (3), the upper limit of the number of binding sites with the partial sequence is 1 or 2.

26. The primer design device according to any one of claims 14 to 25, further comprising:
a communication interface,
wherein the design device is capable of being connected to a server via an external communication network by the communication interface and is capable of operating at least one unit selected from the group consisting of the base sequence data acquisition unit, the target site information acquisition unit, the base conversion unit, the complementary strand generation unit, the partial sequence cutting unit, the primer candidate sequence selection unit, and the primer sequence determination unit by programs in the server.

27. A primer design program,
wherein the primer design program performs the primer design method according to any one of claims 1 to 13 on a computer.

28. A computer-readable recording medium,
wherein the primer design program according to claim 27 is recorded.
